# EUROPÄISCHE PATENTANMELDUNG

(11) **EP 2 586 437 A1**
(43) Veröffentlichungstag der Anmeldung: **01.05.2013**
(21) Anmeldenummer: 12199019.6
(22) Anmeldetag: 01.07.2008
(51) Int. Cl.: A61K 31/198, A61K 31/375, A61K 33/30, A61K 9/00, A61P 17/02

(54) **Zusammensetzung, enthaltend mindestens einen nutritiven, mindestens einen desinfizierenden bzw. dekontaminierenden und/oder mindestens einen proteasen hemmend wirkenden Wirkstoff und/oder Wirkstoffkomplex**

(30) Priorität: 03.07.2007 DE 102007030931
(62) Teilanmeldung aus: 08774571.7
(71) Anmelder: Riesinger, Birgit, 48149 Münster (DE)
(72) Erfinder: Riesinger, Birgit, 48149 Münster (DE)
(74) Vertreter: Michalski Hüttermann & Partner Patentanwälte

(57) **Zusammenfassung**

Die vorliegende Erfindung betrifft eine Zusammensetzung, enthaltend mindestens einen nutritiven, mindestens einen desinfizierenden bzw. dekontaminierenden und/oder mindestens einen Proteasen hemmend wirkenden Wirkstoff und/oder Wirkstoffkomplex zur äußeren Versorgung und/oder Behandlung von Wunden des menschlichen oder tierischen Körpers. (Fig. 1A)

## Beschreibung

Die vorliegende Erfindung betrifft eine Zusammensetzung zur äußeren Versorgung und/oder Behandlung von Wunden gemäß dem Oberbegriff des Anspruchs 1.

### Stand der Technik

Viele Wunden bei Tieren und Menschen, insbesondere chronische, also länger als mehrere Wochen bestehende Wunden, oder Fisteln aufweisende Wunden, weisen in der Regel eine verzögerte Wundheilung auf. Meist muss dabei ein Gewebeverlust durch nachwachsendes Gewebe vom Wundgrund her ersetzt werden. Merkmal solcher Wunden ist die Bildung von pathologischem Exsudat, das eine Schädigung und Verzögerung der Wundheilung bewirkt. Solche Wunden finden sich insbesondere an den Gliedmaßen (insbesondere Ulcus cruris), im Rückenbereich (Dekubitus), aber auch im Mundbereich (dental- und kieferchirurgische Wunden).

Entsprechend der schädigenden Wirkung, die durch das pathologische Exsudat auf die Wunde ausgeübt wird und die damit den Prozess der Chronifizierung weiter voran treibt, ist das Entfernen des pathologischen Exsudats Voraussetzung für eine Progression der Wundheilung. Im Stand der Technik sind daher Wundauflagen mit superabsorbierenden Partikeln oder PU-Schäumen beschrieben, die eine hohe Aufnahmekapazität aufweisen. Diese nehmen Exsudat aus der Wunde auf und binden es. Durch die hohe Saugleistung wird dabei das Exsudat nicht nur oberflächlich, sondern auch aus der Tiefe der Wunde heraus aufgenommen. So werden Wundgrund und Wundrand beispielsweise vor der aktiven Schädigung durch Proteasen besser geschützt. Solche Wundauflagen sind z.B. aus der WO03094813 der Anmelderin der vorliegenden Erfindung bekannt.

Aufgrund ihrer hohen Aufnahmekapazität können diese Wundauflagen länger, d.h. bis zu mehreren Tagen, auf der Wunde verbleiben. Die Wundruhezeit wird also erheblich verlängert und lästige und schmerzhafte Verbandwechsel reduziert. Dies trägt zur Verbesserung der Wundsituation bei, wird doch durch die reduzierte Verbandwechselfrequenz das Auskühlen der Wunde ebenso wie das Infektionsrisiko und die Gefahr sekundärer Wundtraumata verringert.

Die lange Verweildauer kann jedoch zu septischen Prozessen führen, da die Wunde seltener gereinigt und/oder desinfiziert wird. Zumal muss in einigen Fällen damit gerechnet werden, dass sich Mikroorganismen in der Wundauflage vermehren und die Wunde sekundär reinfizieren.

Hinzu kommt, dass insbesondere chronische Wunden unter einer Nährstoffmangelsituation leiden, die die Wundheilung erheblich verzögern und beeinträchtigen.

Diesen genannten Problemen insbesondere chronischer Wunden wird bislang durch die genannten Wundauflagen keinerlei Rechnung getragen

### Offenbarung der Erfindung

Aufgabe der vorliegenden Erfindung ist es daher, eine Zusammensetzung bereitzustellen, die es ermöglicht, septische Prozesse in chronischen, mäßig bis stark exsudierenden Wunden zu begegnen.

Weitere Aufgabe ist es, eine Zusammensetzung bereitzustellen, die es ermöglicht, die Wundheilung chronischer, mäßig bis stark exsudierender Wunden zu fördern.

Weitere Aufgabe ist es, eine Wundauflage bereitzustellen, die eine lange Verweildauer auf der Wunde ermöglicht, ohne dass zu befürchten ist, dass es zu septischen Prozessen kommt.

Weitere Aufgabe ist es, eine Zusammensetzung bereitzustellen, die die Aktivität wundheilungsgefährdender Proteasen reduziert.

Diese Aufgaben werden mit den Merkmalen des vorliegenden Anspruchssatzes gelöst. Die Unteransprüche geben bevorzugte Ausführungsformen an. Dabei ist zu beachten, dass die genannten Bereichsangaben durchweg einschließlich der jeweiligen Grenzwerte zu verstehen sind.

Demnach ist eine Zusammensetzung vorgesehen, enthaltend mindestens einen nutritiven und/oder mindestens einen desinfizierenden Wirkstoff und/oder Wirkstoffkomplex zur äußeren Versorgung und/oder Behandlung von Wunden des menschlichen oder tierischen Körpers.

Unter dem Begriff "Wirkstoffkomplex" soll im Folgenden nicht nur ein Komplex im chemischen Sinne verstanden werden, sondern insbesondere eine Zusammensetzung synergistisch eine Wirkung hervorrufender Wirkstoffe.

In der Literatur sind die Zusammenhänge zwischen dem Ernährungszustand eines Patienten und der Wundheilung bereits beschrieben (Arnold M, Barbul A (2006), "Nutrition and wound healing", Plast Reconstr Surg 117(7 Suppl): 42S-58S). Ebenso wird darauf hingewisen, dass durch Verbesserung des Ernährungszustandes eines Patienten die Wundheilung verbessert werden kann (Patten JA (1995) "Nutrition and wound healing", Compend Contin Educ Dent. 16(2):200, 202-4, 206-8). Allerdings wird in der Literatur als Weg für die Behebung dieser Unterversorgung stets ein systemischer Ansatz empfohlen, d.h. ein Ausgleich der Mangelsituation durch Verbesserung der Ernährungssituation. Topische Ansätze sind bislang in der Literatur hingegen nicht beschrieben.

Gründe hierfür sind, dass die Fachwelt erfahrungsgemäß einen sogenannten "off label use", also die Verwendung von bekannten bzw. sogar trivial erscheinenden Stoffen ohne bekannte Wundindikation in einer Wunde scheut. Auch gibt es diesbezüglich massive rechtliche Probleme.

Hinzu kommt, dass nach herrschender Lehrmeinung eine Wundversorgung mit möglichst wenigen und genau definierten Bestandteilen erfolgen sollte. Hingegen sträubt sich die Fachwelt dagegen, komplexe und ggf. weniger genau definierte Zusammensetzungen in der Wundversorgung zu verwenden.

Hinzu kommt noch der Aspekt, dass Stoffe, die zur Verbesserung des Ernährungszustandes eines Patienten oral verabreicht werden können, nicht unbedingt auch für die topische Behandlung verwendet werden können, da sie z. T. in der Lage sind, allergische und/oder Immunreaktionen hervorzurufen bzw. entzündliche Prozesse auszulösen. Für die topische Wundversorgung dürfen daher nur hypoallergene, pyrogenfreie und ggf. sterile Bestandteile verwendet werden, die natürlich in ihrer Herstellung sehr viel teurer sind.

Aus der WO03055536 ist ein Wundpflegeartikel zur lokalen Schmerzbehandlung in einer Wunde bekannt, der Mittel zur Absorption von Wundexsudaten sowie eine schmerzlindernde Zusammensetzung enthält, wobei die Zusammensetzung ein entzündungshemmendes schmerzstillendes Mittel ist.

Ein Produkt mit diesen Eigenschaften ist auch unter dem Markennamen "Biatain - Ibu" der Firma Coloplast erhältlich. Bei dieser Anmeldung bzw. diesem Produkt steht jedoch der schmerzstillende Aspekt im Vordergrund, nicht der Aspekt des Förderns der Wundheilung, der ungleich komplexer ist als der der Schmerzstillung.

Weiterhin ist erfindungsgemäß die Verwendung einer oder mehrerer solcher Zusammensetzungen zur Herstellung eines Mittels zur äußeren, nicht-systemischen, topischen Versorgung und/oder Behandlung von Wunden des menschlichen oder tierischen Körpers vorgesehen.

Besonders bevorzugt ist dabei vorgesehen, die Darreichungsform des mindestens einen Wirkstoffs oder Wirkstoffkomplexes so zu wählen, dass er lediglich eine topische, nicht systemische Wirkung entfaltet.

Unter dem Begriff "nicht-systemisch" soll im Folgenden verstanden werden, dass der mindestens eine Wirkstoff lediglich eine lokale und/oder topisch applizierte Wirkung im Bereich der Wunde entfaltet. Dies kann z. B. durch geeignete Auswahl der Konzentration und/oder der Darreichungsform des mindestens einen Wirkstoffs erfolgen. Hierauf wird weiter unten noch genauer eingegangen.

Weiterhin ist bevorzugt vorgesehen, dass der mindestens eine Wirkstoff oder Wirkstoffkomplex in einer Form vorliegt, die nach mittelbarer oder unmittelbarer Aufbringung derselben auf die Wunde eine Migration derselben in die Wunde und/oder in die Zellen der Wundregion ermöglicht. Es wird an eine sukzessive, schrittweise Zuführung der wundheilungsfördernden Mittel über die Dauer der Anwendung unter Reduktion der Konzentration dieser Stoffe im Verband und Anreicherung in der Wundregion gedacht.

In einigen Ausführungsformen ist überdies vorgesehen, dass mindestens ein Wirkstoff oder Wirkstoffkomplex in einer Form vorliegt, der eine Aufnahme durch die Zellen der Wundregion ermöglicht.

Unter dem Begriff "unmittelbare Aufbringung" soll verstanden werden, dass die Zusammensetzung direkt, ggf. in pulverförmiger, flüssiger, pastöser oder gelartiger Form auf die Wunde aufgebracht wird.

Unter dem Begriff "mittelbare Aufbringung" soll hingegen verstanden sein, dass die Zusammensetzung z.B. in eine Wundauflage oder in einen anderen Träger eingebracht ist bzw. wird, und letztere bzw. letzterer dann auf die Wunde aufgebracht wird.

Unter dem Begriff "Migration" sollen im Folgenden passive und aktive Transportprozesse verstanden werden, die einen Eintrag des mindestens einen Wirkstoffs oder Wirkstoffkomplexes in die Wunde bewirken. Dies sind insbesondere diffusive Prozesse, aber auch aktive Transportprozesse sowie mechanische Einwirkungen auf eine ggf. vorliegende Wundauflage, die einen Flüssigkeitsaustritt aus der Wundauflage in die Wunde gewährleisten.

Exsudat ist eine über die entzündlichen Prozesse des Wundödems vom Blutplasma abgeleitete Wundflüssigkeit. So wie das Blut für den Transport von Nährstoffen und anderen Botenstoffen und damit für die Versorgung verschiedener Teile des Körpers verantwortlich ist, dient das Exsudat auf ganz ähnliche Weise der Versorgung des Wundbettes und der darin ablaufenden Heilungsprozesse. Um dieser Vielzahl an Funktionen gerecht zu werden, enthält es ein breites Spektrum an Komponenten, woraus ein spezifisches Gewicht resultiert, das leicht oberhalb dessen von Wasser liegt. Darin unterscheidet es sich auch vom Transsudat, welches von nicht-entzündlichen Prozessen abgeleitet ist und ein deutlich geringeres spezifisches Gewicht mit einem geringen Zell- und Proteingehalt aufweist. Neben der Bereitstellung von Nährstoffen für die Fibroblasten und Epithelzellen koordiniert das Exsudat die verschiedenen Prozesse der Wundheilung zeitlich und räumlich durch seinen
hohen Gehalt an Wachstumsfaktoren und Zytokinen. Diese werden vor allem durch Thrombozyten, Keratinozyten, Makrophagen und Fibroblasten gebildet. Sie beeinflussen die Motilität, Migration und Proliferation der verschiedenen an der Wundheilung beteiligten Zellen. So wird das Einwandern von Zellen in den Wundgrund ebenso gefördert wie die Versorgung des neugebildeten Granulationsgewebes durch die Angiogenese. Auch die Wundreinigung wird durch das Exsudat unterstützt. Es enthält verschiedene Serin-, Cystein- und Aspartatproteasen sowie Matrix-Metalloproteasen, die in ihrer Aktivität streng reguliert irreversibel geschädigtes Gewebe abbauen und somit das Wundbett für die nachfolgenden Phasen der Heilung vorbereiten.

Bestandteile des physiologischen Exsudats sind insbesondere Salze, Glucose, Zytokine und Wachstumsfaktoren, Plasmaproteine, Proteasen (insbesondere Matrix-Metalloproteasen), Granulozyten und Makrophagen.

Kommt es nicht innerhalb einiger Wochen zu einer deutlichen Progression des Wundheilungsverlaufs entsprechend der verschiedenen Phasen der Wundheilung, so spricht man von einer chronischen Wunde. Dabei betrachtet man jedoch bereits länger andauernde exsudative Phasen als Komplikation und spricht von einer pathologischen Exsudation, welche zu einer Chronifizierung der Wunde beitragen kann. Die zugrundeliegenden Ursachen sind meist komplex und können durchaus auch systemischer Natur sein. Es überrascht jedoch aufgrund der zuvor erläuterten Bedeutung des Exsudats für die Wundheilung nicht, dass sich Komplikationen der Wundheilung in einer deutlich veränderten Zusammensetzung und Wirkung des Exsudats widerspiegeln. Unter anderem durch eine Konzentrationsverschiebung der einzelnen Bestandteile des Exsudats verliert das normalerweise heilungsfördernde Exsudat bei chronischen Wunden seine positive Wirkung. Insbesondere der Gehalt an inflammatorischen Zytokinen und Proteasen ist in pathologischem Exsudat signifikant erhöht. Der Gehalt an Wachstumsfaktoren ist dagegen verringert.

Ein besonders gravierender Unterschied ergibt sich hinsichtlich der Aktivität der zuvor angesprochenen Proteasen. Neben der Vorbereitung des Wundbetts sind sie auch beim späteren Umbau des Granulations- zum Narbengewebe beteiligt. Diese Enzyme werden normalerweise als inaktives Präenzym gebildet und in ihrer Aktivierung durch entsprechende Inhibitoren reguliert ("tissue inhibitors of metalloproteases, TIMPs"), welche gleichzeitig selbst eine positive Wirkung auf das Zellwachstum haben. Im chronischen Exsudat scheint aufgrund von Störungen in diesem Regulationssystem die Aktivität der Proteasen erhöht, was zu einer aktiven Wundregression beiträgt.

Unter dem Begriff "nutritiver Wirkstoff bzw. Wirkstoffkomplex" sollen im folgenden Makro- und Mikronährstoffe verstanden werden, insbesondere Mineralstoffe, Spurenelemente, Vitamine, Provitamine, Vitaminderivate, Proteine, proteineogene und nicht proteinogene Aminosäuren, Kohlenhydrate, insbesondere Stärke, Glucose und Fructose, Fette, Fettsäuren und fettähnliche Stoffe (insbesondere Mono- und Diglyceride, Phospholipide, Lipide, Cholesterin, Squalen, Carotin), Organische Säuren, Nukleinsäuren und/oder primäre bzw. sekundäre Pflanzenstoffe.

Unter dem Begriff "desinfizierender Wirkstoff bzw. Wirkstoffkomplex" sollen im Folgenden antiseptisch, antibiotisch, bakteriostatisch, virostatisch und/oder antimykotisch wirkende Substanzen verstanden werden. Dabei kann vorgesehen sein, dass die antiseptische, antibiotische, bakteriostatische, virostatische und/oder antimykotische Wirkung lediglich bestimmte Vertreter der jeweiligen Organismenklasse (Bakterien, Viren und/oder Pilze) erfasst, insbesondere pathogene Vertreter, während andere Vertreter, insbesondere nicht pathogene Vertreter, nicht erfasst werden.

Unter dem Begriff "dekontaminierender Wirkstoff bzw. Wirkstoffkomplex" sollen im Folgenden der Dekontamination dienende Wirkstoffe verstanden werden. Eine Dekontamination hat den Zweck, mögliche Schadstoffe von einer Oberfläche zu Entfernen. Die Dekontamination ist erfolgreich verlaufen, wenn die Partikel von der Oberfläche entfernt sind und geeignet entsorgt werden konnten. Bei der Dekontamination der chronischen Wunde ergibt sich für physikalische Möglichkeiten ein nur sehr eingeschränktes Betätigungsfeld. Anforderungen an der Dekontamination der chronischen Wunde umfassen zum einen die Entfernung von Zelldetritus, eingetrocknetem Wundsekret, und gegebenenfalls abgestorbenen Gewebe (Nekrosen). Zum anderen werden im Rahmen der Dekontamination auf diesen Materialien aufsitzende und in diesen Materialien eingeschlossene Mikroorganismen, in aller Regel Bakterien, entfernt. Ziel der Dekontamination ist es, die Keimzahl und die Verschmutzung von der Wunde so gering zu halten, dass sie den natürlichen Heilungsvorgänge nicht behindert werden.

Hintergrund dieses Ansatzes sind - anders als eingangs erwähnt - nicht unbedingt chronische Wunden, sondern auch akute Traumata, wie sie z.B. in militärischen Einsätzen zu beobachten sind, so z.B. durch Verstrahlungen, Verbrennungen oder den Einfluß von biologischen Waffen. Im Zusammenhang mit biologischen Waffen sind insbesondere die desinfizierenden Eigenschaften vorliegend von Bedeutung. Hinzu kommt, dass die im Weiteren beschriebenen Superabsorbierenden Polymere Mikroorganismen, insbesondere auch solche aus biologischen Waffen, aufnehmen und immobilisieren können. Eine Wundauflage enthaltend desinfizierend wirkende Wirkstoffe sowie superabsorbierende Polymere kann daher z.B. in der Erstversorgung biologisch kontaminierter Personen, insbesondere Soldaten, verwendet werden. Sie muss nach Verwendung fachgerecht entsorgt - d.h. in der Regel autoklaviert - werden.

Bei Verbrennungen und Verstrahlungen, die ebenfalls häufig im Zusammenhang mit militärischen Konflikten zu beobachten sind, kommt es hingegen zu Gewebsnekrosen, die teils oberflächlich sein können, teils jedoch tief im Gewebe sitzen und sich mittels Fisteln den Weg zur Oberfläche bahnen können. Solche Wunden sind extrem infektionsgefährdet. Hier kann eine Wundauflage enthaltend eine desinfizierend wirkende Zusammensetzung sowie superabsorbierende Polymere ebenfalls sehr hilfreich sein. Hinzu kommt, dass eine solche Wundauflage imstande ist, nekrotische Bestandteile sowie ggf. strahlende Partikel aus dem Wundbereich - insbesondere über die sich Wege zur Oberfläche hin bahnenden Fisteln - aufzunehmen und zu binden. Auch eine solche Wundauflage muss nach Verwendung fachgerecht entsorgt werden.

Der dekontaminierende Effekt kann dabei durch die Hinzugabe nekrolytischer und/oder exsudationsfördernder Bestandteile, wie sie im Folgenden noch beschrieben werden, bewirkt oder verstärkt werden. Die Förderung der Nekrolyse erleichtert dabei den Abbau und die Aufnahme kontaminierter bzw. kontaminierender Bestandteile, während die Förderung der Exsudation das Auswaschen und damit die Dekontamination erleichtert.

Unter dem Begriff "Proteasen hemmend wirkender Wirkstoff bzw. Wirkstoffkomplex" sollen im Folgenden solche Wirkelemente verstanden werden, die auf Proteasen im Wundbereich, insbesondere Matrix-Metalloproteasen, hemmend wirken. Dies können insbesondere ansäuernd wirkende Wirkstoffe bzw. Wirkstoffkomplexe sein, aber auch Proteasehemmer, superabsorbierende Polymere, Chelatoren für zweiwertige Kationen, Kollagen, oder beschichtete magnetische Partikel.

Unter dem Begriff "ansäuernd wirkender Wirkstoff bzw. Wirkstoffkomplex" sollen im Folgenden solche Wirkelemente verstanden werden, die auf die Wunde eine ansäuernde Wirkung haben, d. h. den pH-Wert der Wunde zu senken im Stande sind. Dies kann sinnvoll sein, um die wie oben erwähnt in bestimmten Phasen schädlich wirkenden Proteasen, deren Aktivitätsmaximum in der Regel im neutralen bis alkalischen Bereich liegt, zu hemmen. Bei dem "ansäuernd wirkenden Wirkstoff bzw. Wirkstoffkomplex" kann es sich daher z. B. um Säuren, insbesondere organische Säuren, handeln. Ebenso kann es sich um einen im sauren wirkenden Puffer handeln. Weiterhin kann es sich um nicht pathogene, säureproduzierende Bakterien, insbesondere Milchsäurebakterien, um Präbiotika, d. h. von solchen säureproduzierenden Bakterien selektiv als Nahrungsgrundlage verstoffwechselbare Substrate handeln, oder um Symbiotika handeln (siehe unten).

Die Wirkstoffe bzw. Wirkstoffkomplexe liegen wie erwähnt bevorzugt in wasserlöslicher Form vor. Dies bedeutet insbesondere auch, dass sie bevorzugt in exsudat-löslicher Form vorliegen.

Je stärker eine Wunde nun exsudiert, desto höher wird aufgrund der oben erwähnten Löslichkeit der Anteil an gelösten und damit der Wunde zugeführten Wirkstoffen. Dies ist besonders vorteilhaft bei einer Vielzahl von Wunden, bei denen der Versorgungsbedarf proportional mit dem Grad der pathologischen Exsudation ansteigt. In diesem Fall stellt sich aufgrund der erfindungsgemäßen Ausgestaltung ein selbstregulierender Mechanismus ein, bei welchem sich die Wunde ihren Bedarf an Wirkstoffen im Sinn der obigen Definition durch den Grad ihre eigenen Exsudation "selbst beschafft".

Es kann dabei vorgesehen sein, dass eine leichte Exsudation zunächst dazu führt, dass die Zusammensetzung selbst in der Funktion eines Absorbens Exsudat aufnimmt, was vorübergehend sinnvoll sein kann. Kommt es über die Zeit zu stärkerer Exsudation, so lösen sich die Bestandteile der Zusammensetzung auf und werden über den Rückfluss in die Wundregion der Wunde zugeführt (Depotwirkung).

In einer anderen Ausgestaltung kann vorgesehen sein, dass die Wirkstoffe bzw. Wirkstoffkomplexe zumindest teilweise in fettlöslicher Form vorliegen. Dies kann z.B. von Vorteil sein, wenn fetthaltige oder unpolare Träger zum Einsatz kommen, wie z. B. Vaseline, die weiter unten beschriebenen erdölbasierenden Zugpräparate, oder eine Fettgaze.

Weiterhin ist erfindungsgemäß vorgesehen, dass der mindestens eine Wirkstoff oder Wirkstoffkomplex in eine wasserlösliche Matrix eingebracht ist. Eine solche Matrix kann z.B. die Form eines wasserlöslichen Sheets aufweisen. Wird diese Matrix auf die Wunde aufgebracht, löst sie sich rückstandsfrei auf und die Wirkstoffe bzw. Wirkstoffkomplexe können ihre Wirkung ausüben. Geeignete Materialien für ein solches Sheet bzw. eine solche Matrix sind z.B. Polysaccharide wie modifizierte (d. h. wasserlösliche) Stärke, mit welcher eine obladenartige Struktur ausgearbeitet wird, oder Gelatine.

Besonders bevorzugt ist vorgesehen, dass der mindestens eine Wirkstoff oder Wirkstoffkomplex in eine flächige Matte eingebracht ist.

Bei dieser Matte kann es sich z. B. um eine Alginatmatte, eine Matte oder Lage aus Carboxymethylcellulose, aus einem Airlaid, aus einem Vlies, aus PU, aus Seide oder aus Zellstoff handeln. Insbesondere kann es sich auch um ein Feuchttuch handeln, das mit einer Lösung enthaltend die erfindungsgemäße Zusammensetzung getränkt ist, handeln. In dieser Ausgestaltung ist in der Regel eine weitere Wundauflage erforderlich, mit deren Hilfe die Matte auf der Wunde fixiert wird. Eine solche Wundauflage ist z.B. in der DE102007019622 der Anmelderin beschrieben, deren Offenbarungsgehalt dem Offenbarungsgehalt der vorliegenden Anmeldung vollumfänglich hinzugefügt sein soll.

Bei dieser Wundauflage kann es sich z.B. um eine Wundauflage zur Absorption von Wundexsudaten handeln, wie sie z.B. in der DE10059439 sowie der WO03094813 der Anmelderin der vorliegenden Erfindung beschrieben ist, deren Inhalt vollumfänglich dem Offenbarungsgehalt der vorliegenden Beschreibung hinzugefügt werden soll. Die Größe der Matte kann in etwa der der Wundoberfläche entsprechen, um der Wunde die besagten Wirkstoffe bzw. Wirkstoffkomplexe möglichst homogen zuzuführen.

Alginat (oder Alginsäure, E400) wird von Braunalgen in den Zellwänden gebildet und stellt in der Alge das strukturgebende Element dar. Alginat findet vor allem als Verdickungs- oder Geliermittel Verwendung. Zur Gelierung kommt es durch Einlagerung von Calciumionen, wobei dreidimensionale Strukturen ausgebildet werden. Weil diese Reaktion mit dem Calcium schlagartig erfolgt, wird in der Praxis mit verschiedenen Methoden gearbeitet, um die Reaktion kontrolliert ablaufen zu lassen. Hierzu werden häufig schwerlösliche Calciumsalze verwendet, die mittels langsamer Säuerung das Calcium nach und nach freisetzen. Außerdem finden auch Sequestranten Verwendung, die ein Teil des Calciums binden können.

Die genannten Wirkstoffe bzw. Wirkstoffkomplexe können bevorzugt auf eine Matte aus Alginaten aufgetragen werden. Die leichte, nicht sehr kapazitätsreiche Quellung von Alginaten ermöglicht eine besonders gute Adaptation an den Wundgrund mit besonders hoher Auflage- und Kontaktfläche zur Wundoberfläche, so dass hier ein besonders intensiver Austausch von Nährstoffen in die Wundregion stattfinden kann. Die Alginatmatte bzw. eine andere wundnahe Materialschicht kann wenigstens einen mäander-, labyrinth-, spiral- oder radialartig verlaufenden Einschnitt aufweisen, der ihre Anpassung an Beschaffenheit des Wundgrundes erleichtern kann.

Carboxymethycellulosen (CMC) sind Derivate der Zellulose, bei denen ein Teil der Hydroxylgruppen der Zellulose als Ether mit einer - CH2 - COOH-(Carboxymethyl-) Gruppe verknüpft sind. Bei der Herstellung wird die Zellulose in reaktivere Alkalizellulose überführt und anschließend mit Chloressigsäure zur Carboxymethylzellulose umgesetzt. Die Cellulose-Struktur bleibt erhalten. In der Säureform sind CMC unlöslich in Wasser. Unter alkalischen Bedingungen sind sie hingegen relativ gut in Wasser löslich.

Matten aus Carboxymethycellulosen ermöglichen eine gute Wundgrundadaptation und weisen eine hohe Affinität zu Wasser auf, dabei ist besonders das Potential des Verdrängens anderer Stoffe in der CMC-Matte durch Wasser hervorzuheben. Auf diese weise werden Wirkstoffe bzw. Wirkstoffkomplexe, die an die Wundoberfläche abgegeben werden sollen, insbesondere bei Übersättigung mit Exsudat aus der Matte herausgewaschen und so verfügbar gemacht.

PU-Matten sind geschäumte Auflagen aus Polyurethan, die die Eigenschaft haben, auf der Basis von Kapillarkräften Wasser aufzunehmen und sich sodann auszudehnen. Wenn diese die besagten Wirkstoffe bzw. Wirkstoffkomplexe enthalten, führt die niedrige Retentionskraft des Schaums bei Benetzung, beispielsweise durch Exsudat, zum Auswaschen der Wirkstoffe bzw. Wirkstoffkomplexe in die Wunde.

Ein Vliesstoff ist ein textiles Flächengebilde, das im Gegensatz zu aus Garnen hergestellten Geweben, Gestricken und Gewirken aus einzelnen, nicht verwobenen Fasern ("non woven") besteht. Ein Vliesstoff unterscheidet sich vom Papier häufig in der Länge der Fasern, welche dort sehr viel kürzer sind. Vliese weisen häufig ein gutes Aufnahmevermögen für Flüssigkeiten auf.

Häufig werden die genannten Materialien als Airlaids hergestellt. Dieses Verfahren führt zu sehr weichen und saugfähigen Produkten mit häufig dreidimensional strukturierter Oberfläche, die sich besonders für die Verwendung mit der erfindungsgemäßen Zusammensetzung eignen.

Die erfindungsgemäße Zusammensetzung kann bevorzugt auf einen Träger aus Seide und/oder Hydrofasern aufgebracht und/oder in Fettgaze eingeschlagen, in Kolloid eingebettet, in Silikon eingebettet, in CMC eingebettet, in Polyesterfolie eingebettet und/oder in Polyethylenfolie eingebettet vorliegen.

Seide ist eine feine Textilfaser, die aus den Kokons der Seidenraupe, der Larve des Seidenspinners, gewonnen wird. Aufgrund ihrer hygroskopischen Eigenschaften in fließend liegender Vlieslage kann die Wirkstoffabgabe in die Wundregion verbessert werden.

Hydrofasern werden aus Natrium-CMC und Polyesterfasern hergestellt und können große Mengen an Flüssigkeiten speichern. Die Feuchtigkeit wird in die Fasern aufgenommen und breitet sich dadurch horizontal kaum aus, so dass die Gefahr von Mazeration sehr minimal ist. Unter den Wundauflagen bildet sich ein feucht-warmes, wundheilungsförderliches Millieu. Sie eignen sich in Zusammenhang mit der erfindungsgemäßen Zusammensetzung zum Exsudatmanagement und zur Förderung der Granulation.

Fettgaze ist eine Gaze, die zur Verhinderung des Verklebens mit der Wundregion mit einer fettartigen Substanz wie Vaseline, Wachs oder Fett, versehen wird.

Überdies eignet sich die Beschichtung als Speicher für fettlösliche Wirkstoffe und/oder Wirkstoffkomplexe im Sinne der Erfindung. Diese werden bei Kontakt mit der Wundregion sukzessive freigesetzt und der Wunde zugeführt.

Bei einem Kolloid handelt es sich um ein System aus Clustern oder um kleine Festkörper, die innerhalb eines Mediums fein verteilt vorliegen. Die Teilchen dieser so genannten kolloid-dispersen Phase weisen in der Regel Größenordnungen von 1 bis 1000 nm in mindestens einer Dimension auf, das Medium selbst bezeichnet man als Dispersionsmedium. Der Bereich der Chemie, der sich mit Kolloiden befasst, ist die Kolloidchemie. Eine Gruppe bilden Dispersionen einer Flüssigkeit in einer anderen Flüssigkeit, insofern beide nicht miteinander mischbar sind. Beispiele für diese so genannten Emulsionen sind zahlreiche Kosmetika. Handelt es sich um mehr als zwei Stoffe, also so genannte multiple Kolloide, spricht man analog von multiplen Emulsionen.

Kolloide weisen eine enorm große innere Oberfläche auf, was insbesondere bei Wirkstoffen, die eine Kontaktwirkung aufweisen (so z.B. antibakteriell wirkende kolloidale Silberionen) von großem Vorteil ist. Technologien zur Herstellung solcher Kolloide fallen dem Bereich der Nanotechnologie zu.

Silikon ist eine Bezeichnung für eine Gruppe synthetischer Polymere, bei denen Siliziumatome über Sauerstoffatome zu Molekülketten und/oder netzartig verknüpft sind. Die restlichen freien Valenzelektronen des Siliziums sind dabei durch Kohlenwasserstoffreste (meist Methylgruppen) abgesättigt. Silikone gelten in der Regel als physiologisch verträglich (nicht gesundheitsschädlich), weshalb sie für den Hautschutz, die kosmetische Hautpflege und plastische Chirurgie genutzt werden.

Polyester (PE) sind Polymere mit Esterbindungen -[-CO-O-]- in ihrer Hauptkette. Sie können im Rahmen der vorliegenden Erfindung als inerte Träger ohne Rückhaltekraft für Wirkstoffe und/oder Wirkstoffkomplexe verwendet werden.

Polyethylen (PET) ist ein durch Polymerisation von Ethen [CH2 = CH2] hergestellter thermoplastischer Kunststoff. Folien aus PET können im Rahmen der vorliegenden Erfindung als inerte Träger ohne Rückhaltekraft für Wirkstoffe und/oder Wirkstoffkomplexe verwendet werden. Zu diesem Zweck können auch andere thermoplastischen Kunststoffe, wie Polyester oder Polyamide, geeignet sein.

In einer anderen bevorzugten Ausgestaltung ist vorgesehen, dass der mindestens eine Wirkstoff oder Wirkstoffkomplex in ein Wunddistanzgitter eingebracht ist.

Ein Wunddistanzgitter hat unmittelbaren Kontakt zu einer Wunde und verhindert, das ein auf das Wunddistanzgitter aufzulegender Sekundärverband (z.B. eine Wundauflage enthaltend Superabsorber) mit der Wunde verklebt. Eine solches Wunddistanzgitter aus Polyethylen oder Polyester - jedoch ohne die erfindungsgemäße Zusammensetzung - ist z. B. unter dem Handelsnamen "Sorbion Plus" bekannt. Der Vorteil dieser Ausgestaltung ist, dass das Winddistanzgitter unmittelbaren Kontakt zur Wunde hat und die Wirkstoffe der Zusammensetzung daher direkt an den Wirkort gebracht werden können, lange Diffusionswege somit vermieden werden. Weitere vorteilhafte Ausgestaltung eines solchen Distanzgitters sind z.B. Fette, (Lipo-) Kolloide, Silikon oder Carboxymethylcellulose enthaltende Gazen. Aus der DE102006017194 der Anmelderin der vorliegenden Erfindung, deren Offenbarungsgehalt dem Offenbarungsgehalt der vorliegenden Anmeldung vollumfänglich hinzugefügt sein soll, ist bereits ein solches Wunddistanzgitter bekannt. Dieses besteht aus einer Folie aus einem Thermoplasten, die eine Vielzahl von dreidimensionalen Perforationen aufweist, deren Wände von der ersten, glatten Oberfläche beginnend jeweils in einen Randüberstand mit freier Kante auslaufen und der zweiten Oberfläche einen rauen Griff verleihen. Dieses Distanzgitter weist auf seiner einen Seite Kavitäten auf, in welchen Wirkstoffe der Zusammensetzung vorkonfektioniert angeordnet sein und so direkt an den Wirkort gebracht werden können.

Ebenso kann vorgesehen sein, dass der mindestens eine Wirkstoff oder Wirkstoffkomplex in eine absorbierende Wundauflage aufweisend mindestens ein Wirkelement ausgewählt aus der Gruppe enthaltend eine Schaumstoffmatte, ein Airlaid, eine Carboxymethylcellulosematte, eine Alginatmatte und/oder eine Matte aufweisend superabsorbierende Partikel eingebracht ist.

Bei besagter Schaumstoffmatte kann es sich z. B. um eine Matte aus Polyurethanschaum handeln.

Superabsorbierende Partikel (auch superabsorbierende Polymere genannt) sind Kunststoffe, die in der Lage sind, ein Vielfaches ihres Eigengewichts an Flüssigkeiten aufzunehmen. Das Produkt kommt als weißes, grobkörniges Pulver mit Partikelgrößen von 100 - 1.000 µm (= 0,1 - 1,0 mm) zum Einsatz. Es findet größtenteils in Babywindeln, jedoch auch in Produkten für die Damenhygiene und der Inkontinenzversorgung. Chemisch handelt es sich bei Superabsorbern in der Regel um Copolymere aus Acrylsäure (Propensäure, C₃H₄O₂) und Natriumacrylat (Natriumsalz der Acrylsäure, NaC₃H₃O₂), wobei das Verhältnis der beiden Monomere zueinander variieren kann. Zusätzlich wird ein so genannter Kernvernetzer (Core-Cross-Linker, CXL) der Monomerlösung zugesetzt, der die gebildeten langkettigen Polymermoleküle stellenweise untereinander durch chemische Brücken verbindet (sie "vernetzt").

Diese Wundauflage weist bevorzugt 10-60 Gew.-% dieser Superabsorber (SAP) auf. Sie ist bevorzugt so ausgestaltet, dass sie keine Bindemittel aufweist. Hierzu ist bevorzugt vorgesehen, dass der Kern der Wundauflage aus geschnittenen Zellulosefasern bzw. -lagen besteht, die über Verkantungen mit den SAP-Partikeln in Verbindung stehen und der Wundauflage so Stabilität geben.

Bevorzugt weist diese Wundauflage beidseitig dieses Kerns zwei Zellulosematten auf, sowie ggf. eine Hülle aus flüssigkeitsdurchlässigem Material.

Eine Wundauflage enthaltend superabsorbierende Partikel ist z. B. in der DE10059439 oder in der EP1507498 der Anmelderin der vorliegenden Erfindung beschrieben, deren Inhalt vollumfänglich dem Offenbarungsgehalt der vorliegenden Anmeldung hinzugefügt sein soll.

Bei einer solchen Wundauflage handelt es sich um einen Absorptionskörper zum Anschluss an den menschlichen Körper, insbesondere zum Aufsaugen von Flüssigkeiten, welche aus menschlichen Körperstellen, wie Wunden, austreten. Dieser Absorptionskörper weist auf einen im wesentlichen flachen Materialabschnitt aus Absorptionsmaterial, bestehend aus einem aufsaugenden Vlies mit darin verteilten Superabsorber-Teilchen, und eine flüssigkeitsdurchlässige Hülle, die den Materialabschnitt umgibt und eine Barriere gegen feste Ausscheidungen bildet und die den Durchtritt von anderen ausgetretenen Substanzen zu dem innerhalb der Hülle angeordneten Materialabschnitt aus Absorptionsmaterial ermöglicht. Eine solche Wundauflage ist z.B. unter dem Markennamen "sorbion sachet" der Firma Sorbion, Deutschland, erhältlich.

Sie eignet sich hervorragend zur hydroaktriven Therapie chronischer Wunden, bei welcher schädliche Exsudate einschließlich der darin enthaltenen schädlichen Bestandteile (insbesondere pathogene Erreger und Proteasen) aufgenommen und von den Superabsorbern eingeschlossen werden, und gleichzeitig ein wundheilungsförderndes Feuchtklima erzeugt wird. Darüber hinaus können solche Wundauflagen aufgrund ihrer hohen Aufnahmekapazität lange auf der Wunde verbleiben, und so ein häufiger Verbandwechsel mit damit einhergehenden Traumata verhindert werden. Die genannten Wundauflagen zeigen gerade bei Behandlung stark exsudierender, chronischer Wunden hervorragende Erfolge.

Die zuvor genannten Wundauflagen können auch so ausgestaltet sein, dass das eigentliche absorbierende Material in Form von Pulver, Granulat, Fetzen, Kugeln oder Schnipseln vorliegt. Auf diese Weise wird die Kontaktfläche zwischen Exsudat und absorbierendem Material vergrößert, und damit einerseits die Aufnahme von Exsudat sowie die Abgabe der Wirkstoffe bzw. Wirkstoffkomplexe der erfindungsgemäßen Zusammensetzung an die Wunde beschleunigt.

Ebenso kann die Wundauflage, eine etwaige Matte oder partikuläre Bestandteile derselben eine konkave Form aufweisen.

Besonders bevorzugt ist eine Ausgestaltung, bei welcher der mindestens eine Wirkstoff oder Wirkstoffkomplex in eine Wundauflage enthaltend superabsorbierende Partikel sowie mindestens eine Schaumstoffmatte und/oder eine Carboxymethylcellulosematte eingebracht ist.

Eine Wundauflage in verschiedenen Ausgestaltungen dieser Konfiguration ist z.B. in der Anmeldung WO2007051599 der Anmelderin der vorliegenden Erfindung beschrieben, deren Inhalt vollumfänglich dem Offenbarungsgehalt der vorliegenden Anmeldung hinzugefügt sein soll. Dasselbe gitl für die DE 20200601682 der Anmelderin der vorliegenden Erfindung.

Ebenso ist die Kombination einer Wundauflage aufweisend superabsorbierende Polymere und eine erfindungsgemäße Zusammensetzung mit einer Vakuum-Drainagevorrichtung vorgesehen. Solche Wundauflagen - noch ohne die erfindungsgemäße Zusammensetzung - sind insbesondere aus den Patentanmeldungen WO2006048240 und WO2006056294 der Anmelderin der vorliegenden Patentanmeldung bekannt.

Grundsätzlich steht bei solchen Wundauflagen, die in der Regel luftdicht mit der Wunde in Verbindung stehen und an eine Vakuumpumpe angeschlossen sind, der Drainageeffekt im Vordergrund. Allerdings ist die Vakuumpumpe nicht kontinuierlich in betrieb, sondern sie wird von zeit zu Zeit abgeschaltet. In diesen Phasen können die wundheilungsfördernden Effekte der erfindungsgemäßen Zusammensetzung zum Tragen kommen.

So kann z.B. eine Sandwichkonstruktion aus einer Zellulosematte enthaltend superabsorbierende Partikel mit relativ hohem Absorptionsvermögen sowie einer Schaumstoffmatte mit relativ geringem Absorptionsvermögen vorgesehen sein. Eine solche Wundauflage läßt eine Anpassung des Absorptionsvermögens auf den Wundtyp bzw. den Exsudationsgrad der Wunde zu, einfach durch Auswahl der geeigneten Seite.

Durch diese Maßnahme kann dem recht häufigen Phänomen der Wundmazeration vorgebeugt werden, das Auftritt, wenn eine Wundauflage mit geringem Absorptionsvermögen (wie z.B. eine reine Schaumstoffmatte) mit Exsudat übersättigt wird.

Insbesondere kann überdies vorgesehen sein, dass ggf. vorgesehene desinfizierend wirkende Stoffe zumindest teilweise so ausgestaltet sind, dass sie in der Wundauflage verbleiben. Auf diese Weise kann ein Bakterienwachstum in der Wundauflage durch im aufgenommenen Exsudat enthaltene Bakterien verhindert werden. Letzterer Prozess spielt insbesondere deswegen eine große Rolle, weil einige der genannten Wundauflagen - insbesondere solche Wundauflagen, die Superabsorber enthalten - große Mengen an Flüssigkeiten aufnehmen und daher lange Zeit auf der Wund verbleiben können. Da die Wundauflage steril ist und erfindungsgemäß nutritive Substanzen enthält, müßte ansonsten mit einem verstärkten Bakterienwachstum in der Wundauflage gerechnet werden.

Weiterhin kommt dem Exsudat in den genannten Ausführungsformen eine Doppelrolle zu. Einerseits handelt es sich in vielen Fällen um eine entfernungspflichtige Flüssigkeit, deren Aufnahme durch die Wundauflage die Wundheilung erheblich fördert. Andererseits dient das Exsudat als Lösungsmittel für die genannten Wirkstoffe bzw. Wirkstoffkomplexe, und trägt so dazu bei, dass diese gelöst und zumindest teilweise in die Wunde geschwemmt werden bzw. dahin diffundieren. Letzterer Prozess spielt insbesondere deswegen eine große Rolle, weil einige der genannten Wundauflagen - insbesondere solche Wundauflagen, die Superabsorber enthalten - große Mengen an Flüssigkeiten aufnehmen und daher lange Zeit auf der Wund verbleiben können. Daher ist in vielen Fällen genügend Zeit gegeben für diffusive Prozesse.

Durch eine verzögerte Löslichkeit der genannten Wirkstoffe bzw. Wirkstoffkomplexe kann so eine Depotwirkung, d. h. eine Versorgung der Wunde über einen längeren Zeitraum, ermöglicht werden.

In einer weiteren, alternativen Ausführungsform ist vorgesehen, dass der mindestens eine Wirkstoff oder Wirkstoffkomplex in eine Lösung eingebracht ist.

Eine solche Lösung kann beispielsweise zum definierten Anfeuchten von Wundauflagen verwendet werden, insbesondere von solchen, die superabsorbierende Partikel enthalten. Auf diese Weise wird einerseits ein wundheilförderndes Feuchtklima erzeugt, andererseits werden die erwähnten nutritiven und/oder desinfizierenden Funktionen wahrgenommen. Auch hier kann die auf diese Weise angefeuchtete Wundauflage eine Depotwirkung wahrnehmen, d.h. eine sukzessiv erfolgende Versorgung der Wunde über einen längeren Zeitraum gewährleisten.

Als Lösemittel kommt hier insbesondere Wasser, aber z.B. auch physiologische Kochsalzlösung, Ringerlösung, Blutserum oder Blutplasma in Frage.

Zwar sind Wundauflagen bekannt, die superabsorbierende Partikel enthalten und mit einer physiologischen Lösung getränkt als Reinigungsauflage für Wunden verwendet werden. Bei besagter Lösung handelt es sich jedoch um eine sogenannte Ringerlösung, die in ihrer Elektolytzusammensetzung derjenigen des Plasmas entspricht (d.h. 8,6 g NaCl, 0,30 g KCl und 0,33 g CaCl₂). Hierbei handelt es sich also nicht um eine Lösung mit nutritiven oder gar desinfizierenden Eigenschaften, da die gewählten Stoffmengen sehr gering sind und in ihrer Zusammensetzung unzureichend sind. Hinzu kommt, dass in Zusammenhang mit solchen Wundauflagen die Verwendung von topischen Medikamenten oder Desinfektionsmitteln ausdrücklich abgeraten wird.

Überdies steht bei diesen Wundauflagen der Spülcharakter im Vordergrund, und nicht der erfindungsgemäß definierte Versorgungscharakter.

Bevorzugt ist weiterhin vorgesehen, dass der mindestens eine Wirkstoff oder Wirkstoffkomplex in eine Creme, Salbe, Milch, ein Gel, eine Suspension, Emulsion und/oder Dispersion eingebracht ist.

In einer ebenfalls bevorzugten Ausgestaltung der vorliegenden Erfindung ist vorgesehen, dass mindestens ein Wirkstoff in einer durch ein Verfahren ausgewählt aus der Gruppe enthaltend Gefriertrocknen, Lyophilisation, Sprühtrocknen, Walzentrocknen und/oder Vakuumverdampfen aufbereiteten Form vorliegt.

Diese Arten der Aufbereitung führen zu Produkten, die sich bei Kontakt mit Flüssigkeit rückstandsfrei auflösen. Solche Produkte sind auch als "Instant"-Produkte bekannnt.

Bevorzugt ist weiterhin vorgesehen, dass die Zusammensetzung bzw. ihre Bestandteile in sterilisierter Form vorliegen.

Dies kann z.B. erfolgen durch Behandlung der Zusammensetzung mit Ethylenoxid und/oder Gammastrahlung. Ebenso ist eine getrennte Sterilisierung der Wundauflage (z.B. mit Ethylenoxid) und der Zusammensetzung (z.B. durch autoklavieren) und späteres Zusammenbringen beider Komponenten unter sterilen Bedingungen denkbar.

Besonders bevorzugt ist erfindungsgemäß vorgesehen, dass es sich bei einem der desinfizierend wirkenden Wirkstoffkomplexe um eine Zusammensetzung aus mindestens einem Vitamin oder Vitaminderivat, einem Metallion sowie einem Detergenz handelt. Bei dieser Zusammensetzung handelt es sich um ein bevorzugtes Beispiel für den zuoberst definierten "desinfizierenden Wirkstoffkomplex".

Eine solche Zusammensetzung ist stofflich z.B. in der W02006116983 bereits beschrieben. Jedoch ist darin nur die Verwendung dieser Zusammensetzung als Dekontaminationslösung für Oberflächen beschrieben, nicht jedoch die Verwendung zur Wunddesinfektion. Nun ist dem Fachmann bekannt, dass sich Agenzien, die für die Oberflächendesinfektion geeignet sind, nicht zwangsläufig auch für die Wunddesinfektion eignen. Ein Beispiel hierfür sind z.B. 70 %-iger Ethanol, Desinfektionsmitttel auf Phenolbasis, oder auf Strahlung oder Temperatureinwirkung basierende Desinfektionsverfahren. In der vorliegenden Erfindung sind erstmals und überraschend die Wirksamkeit dieser Verbindungen für die Wunddesinfektion aufgezeigt.

Dies ist insbesondere deswegen überraschend, da für den Fachmann die Effektivität einer Wirkstoffkombination aus einem Vitamin oder Vitaminderivat, einem Metallion sowie einem Detergenz für die Wunddesinfektion nicht ohne weiteres erschließt. So weist jede dieser Komponenten für sich gesehen keine desinfizierende Aktivität auf. Hinzu kommt, dass auch hier Fachleute den "offlabel use" einer für andere Verwendungszwecke bestimmten Substanz scheuen.

Die Zusammensetzung weist bevorzugt einen pH-Wert im Bereich von 2 bis 8,5 auf. Diese Wirkstoffkombination führt zur selektiven Inaktivierung von Mikroorganismen. Besonders bevorzugt ist vorgesehen, dass die Mischung einen pH-Wert im Bereich von 3 bis 7, vorzugsweise von 4 bis 6, aufweist. Bevorzugt weist diese Zusammensetzung außerdem ein Puffersystem mit Carbonaten und Derivaten der Bernsteinsäure auf. Bei der Verwendung dieses Puffersystems in der erfindungsgemässen Dekontaminationslösung kann der pH-Wert der Lösung, der sich aufgrund der gelösten Komponenten, insbesondere der sauren Vitamine, im stark sauren Bereich befindet, leicht bis beispielsweise in den neutralen oder schwach basischen Bereich angehoben werden, ohne dass die gelösten Metallionen ausfallen.

Die enthaltenen Vitamine bzw. deren Salze oder sauren Derivate sind bevorzugt ausgesucht aus der Gruppe der wasserlöslichen Vitamine mit antioxidativen Eigenschaften, wie vorzugsweise Vitamin C, Riboflavin und Niacin.

Besonders bevorzugt ist dabei insbesondere die Kombination aus Vitamin C, Vitamin E bzw. Vitamin B12. Eine solche Kombination weist besonders synergistische Effekte in Bezug auf die desinfizierende Wirkung auf.

Bei den enthaltenen Metallionen handelt es sich bevorzugt um 2- und/oder 3- wertige Ionen der Metalle der 4. Periode und/oder Nebengruppen I, II und VIII des periodischen Systems der Elemente. Sie werden in Form ihrer Salze mit organischen und/oder anorganische Säuren oder Basen eingesetzt. Besonders bevorzugt sind ein oder mehrere Verbindungen ausgesucht aus der Nebengruppe VIII, insbesondere Eisen, Kobalt, Nickel, Kupfer oder Zink.

Bei den enthaltenen Detergenzien handelt es sich bevorzugt um anionische, nichtionische, amphotere oder kationische Tenside oder geeignete Mischungen miteinander oder untereinander sein. Insbesondere können Alkylethersulfate, Alkyl- und/oder Arylsulfonate, Alkylsulfate, Amphotenside, Betaine, Alkylamidoalkylamine, Alkylsubstituierte Aminosäuren, alkylsubstituierte Iminosäuren, Acylierte Aminosäuren, Amphotensidkombinationen eingesetzt werden. Besonders bevorzugt sind anionische und nichtionische Tenside.

Die genannte Wirkstoffkombination kann als die oder zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein.

Weitere bevorzugt vorgesehene desinfizierend wirkende Wirkstoffe bzw. Wirkstoffkomplexe sind die sogenannten BLIS (engl.: bacteriocin like inhibitory substances). Hierbei handelt es sich um Eiweißmoleküle mit antibiotischen Eigenschaften, die von einigen Bakterienarten produziert werden. Sie können im Rahmen einer "bacterial replacement therapy" nützlich insbesondere gegen einige Streptokokkenarten sein. Beispiele für solche BLIS sind z.B. Colicine aus Colibakterien, Nisin aus *Lactococcus lactis* oder das BLIS aus *Streptococcus salivarius* K12. Ebenso kann es sich bei dem desinfizierend wirkenden Wirkstoff auch um solche BLIS freisetzenden Mikroorganismen handeln, insbesondere um das Bakterium *Streptococcus salivarius* K12. Diese können z.B. in getrockneter Form vorliegen (ähnlich den käuflich erhältlichen getrockneten Joghurtkulturen), dergestalt, dass sie bei Kontakt mit Feuchtigkeit, insbesondere Exsudat, aktivierbar sind.

In diesem Zusammenhang kann auch vorgesehen sein, in der Zusammensetzung rekombinante Mikroorganismen einzusetzen, denen auf rekombinatem Wege die Eigenschaft verliehen wurde, BLIS oder ähnliche Hemmstoffe gegen pathogene Mikroorganismen zu bilden und freizusetzen.

Sowohl die BLIS als auch ggf. getrocknete Mikroorganismen können als die oder zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein.

Weiterhin kann Octenidin bzw. Octenidindihydrochlorid als desinfizierend wirkender Wirkstoff bzw. Wirkstoffkomplex Verwendung finden. Octenidin bzw. Octenidindihydrochlorid wirkt bakterizid sowohl gegen gram-positive wie -negative Keime, viruzid gegen lipophile Viren wie Herpes simplex-und Hepatitis B-Viren. Außerdem wirkt es fungizid, nicht allerdings gegen Sporen. Es ist geruch- und farblos, erlaubt eine schmerzfreie Anwendung, hat ein breites Wirkungsspektrum und eine Remanenzwirkung und ist daher in Zusammenhang mit der erfindungsgemäßen Zusammensetzung besonders geeignet.

Ebenso sind Peroxyde, wie Wasserstoffperoxid sowie auf Wasserstoffperoxid basierende Substanzen in Zusammenhang mit der erfindungsgemäßen Zusammensetzung als desinfizierende Wirkstoffe bzw. Wirkstoffkomplexe geeignet.

Weiterhin können auch andere Peroxide als desinfizierend wirkender Wirkstoff bzw. Wirkstoffkomplex Verwendung finden. Hierbei ist vor allem an Salze der Monoperphthalsäure, vorzugsweise die Substanz Magnesiummonoperphthalat (MMPP), gedacht.

Weiterhin kann Polihexanid als desinfizierend wirkender Wirkstoff bzw. Wirkstoffkomplex Verwendung finden. Polihexanid ist ein kationisches Diguanidin mit breiter mikrobiozider Wirksamkeit, jedoch nicht viruzid oder sporozid wirksam. Aufgrund der guten Gewebeverträglichkeit ist Polihexanid bei empfindlichen und schlecht heilenden chronischen Wunden besonders geeignet, auch bei längerfristiger Anwendung bei Lavagen sowie unter semiokklusiven oder okklusiven Verbänden, z. B. zum Feuchthalten von Wunden, und ist daher in Zusammenhang mit der erfindungsgemäßen Zusammensetzung besonders geeignet.

Weiterhin kann Zinkoxid als desinfizierend wirkender Wirkstoff bzw. Wirkstoffkomplex Verwendung finden. Zinkoxid weist eine antiseptische Wirkung auf und ist daher ebenfalls als desinfizierend wirkender Wirkstoff geeignet.

Weitere in Frage kommende desinfizierend wirkende Wirkstoffe bzw. Wirkstoffkomplexe sind in der Hauptgruppe 33.B.1 der Roten Liste aufgeführt, und sollen sämtlich dem Offenbarungsgehalt der vorliegenden Erfindung hinzugerechnet werden. Insbesondere ist hier an Ethanol (60-90%), 1-Propanol (60-70%) und Isopropanol (70-80%), Borsäure, Chlorhexidin-Gluconat, Iodtinktur, Logolsche Lösung, Iodopovidone/PVP-I, Mercurochrom, 2-phenoxyethanol, Phenol (Karbolsäure), Thymol, Hexachlorophen, Triclosan, Dibromol, und/oder Natriumhypochlorit gedacht.

Weitere in Frage kommende desinfizierend wirkende Wirkstoffe bzw. Wirkstoffkomplexe sind insbesondere Silber, Silberkolloide, Silberhaltige Materialien bzw. Materialien, die die Fähigkeit aufweisen, Silberionen freizusetzen, Peroxide, Gemische und/oder Legierungen aus Silber und Kupfer in einem organischen Träger, die ggf. mit mindestens einem Katalysatoren ausgewählt aus der Gruppe enthaltend Platin, Platindioxid, Titan, Titanoxid und Titandioxid angereichert sind, antibakterielle Bestandteile des Bienenhonigs wie Propolis, Honigenzyme, Gelee Royale etc., Octeinidin, Antibiotika, Antimykotika wie Nystatin, Griseofulvin, Imidazole und deren Derivate wie z.B. Clotrimazol oder Tolnaftat, Virostatika und dergleichen.

Besagte Gemische und/oder Legierungen aus Silber und Kupfer in einem organischen Träger, die ggf. mit mindestens einem Katalysator ausgewählt aus der Gruppe enthaltend Platin, Platindioxid, Titan, Titanoxid und Titandioxid angereichert sind, sind in Zusammenhang mit der desinfizierenden Behandlung von Mundschleimhäuten beschrieben. Besagte Oxide können die Wirkung der Zusammensetzung katalysieren, da sie naszierenden Sauerstoff bilden, der mit dem Silber ein Silberoxidradikal bildet, das in Bezug auf die desinfizierende Wirkung äusserst wirksam ist.

Im Sinne dieses Anspruchs kann auch ein Naturschwamm als Zusammensetzung enthaltend mindestens einen desinfizierend wirkenden Wirkstoff bzw. Wirkstoffkomplex verstanden werden. Solche Naturschwämme beispielsweise der Klasse der Hornkieselschwämme (Demospongiae) weisen wachstumshemmende Eigenschaften gegenüber Mikroorganismen auf, um sich vor dem Ansiedeln von sessilen Organismen zu schützen. Diese Eigenschaften können auch in Zusammenhang mit der Wundversorgung sinnvoll sein, um Bakterienwachstum in der Wundauflage und/oder in der Wunde zu verhindern. Ebenso weisen diese Schwämme wachstumshemmende Eigenschaften gegenüber Pilzen und Einzellern auf. Hinzu kommt, dass solche Schwämme Flüssigkeiten zu absorbieren imstande sind und sich daher für die Aufnahme von Exsudaten hervorragend eignen.

Besagter Naturschwamm kann in dünnen Scheiben, die z. B. durch thermisches Schneiden erzeugt worden sind, auf die Wunde aufgelegt sein. Er kann ebenso in eine bestehende Wundauflage, wie sie z.B. aus den Offenlegungsschriften EP1411874, EP1507498 oder DE202006016821U der Anmelderin der vorliegenden Erfindung bekannt ist, eingearbeitet sein. Ebenso kann vorgesehen sein, dass besagter Naturschwamm mit superabsorbierenden Polymeren versehen ist. Dies ist insbesondere deswegen vorteilhaft, weil so die Bindungskapazität für Wundexsudat weiter erhöht werden kann und gleichzeitig die wachstumshemmenden Eigenschaften des Schwamms ein Keimwachstum im aufgenommenen Exsudat unterbinden.

Ebenso kann es sich bei der Zusammensetzung enthaltend mindestens einen desinfizierend bzw. dekontaminierend wirkenden Wirkstoff bzw. Wirkstoffkomplex auch um die bereits beschriebenen superabsorbierenden Polymere handeln. Werden diese mit einer Bakterien enthaltenden Lösung wie z.B. einem pathologischen Substrat getränkt, so binden sie insbesondere die Bakterien und verhindern so eine Rekontamination der Wunde durch die Wundauflagen. Hier kommen insbesondere elektrostatische Wechselwirkungen zwischen den Polymeren und den Bakterienzellwänden zum Tragen.

Ebenso kann es sich bei der Zusammensetzung enthaltend mindestens einen desinfizierend bzw. dekontaminierend wirkenden Wirkstoff bzw. Wirkstoffkomplex um einen oder mehrere exsudationsfördernde Stoffe handeln. Hierunter werden solche Stoffe verstanden, die die Wundexsudation fördern. Die Förderung der Exsudation ist insbesondere bei kontaminierten und/oder infizierten Wunden angezeigt, bei welchen eine erhöhte Exsudation zu einer Verbesserung des Ausspülens von Keimen und anderen Kontaminanden und damit zu einer Dekontamination führt. Insbesondere im Zusammenhang mit chemischen, biologischen und nuklearen Kontaminationen ist dies sehr vorteilhaft.

Hierbei kann es sich z.B. um die hier beschriebenen erdölbasierenden Zugpräparate handeln, wie z.B. Schieferölsulfonate, aber auch um die ebenfalls beschriebenen Hygroskopika.

Besonders bevorzugt sind hier außerdem Kombinationen aus einem oder mehreren Hygroskopika sowie einem oder mehreren Detergenzien vorgesehen. Diese Kombination kann z. B. aus Glyerin als Hygroskopikum und einem nichtionischen Detergenz wie z. B. Pluronic F68 bestehen. Besagte Bestandteile können in eine Wundauflage eingebracht sein, die wenn sie auf die Wunde aufgelegt wird, die besagten Bestandteile freigibt, so die Wundexsudation fördert und damit die Dekontamination begünstigt.

Weiterhin kann als Zusammensetzung enthaltend mindestens einen desinfizierend bzw. dekontaminierend wirkenden Wirkstoff bzw. Wirkstoffkomplex auch ein Gemisch enthaltend magnetische Partikel ("beads") verstanden werden, die mit einer funktionalisierten Oberfläche versehen sind. Besagte funktionalisierte Oberfläche kann z.B. Antikörper gegen Oberflächenantigene bestimmter pathogener Keime aufweisen. Ebenso kann besagte Oberfläche Agenzien aufweisen, die Kontaminanden, wie z.B. bakterielle Toxine, Zelltrümmer und dergleichen spezifisch oder unspezifisch binden.

Diese Beads können z.B. in einer Lösung vorliegen und so in die Wunde eingebracht werden ("passive Einbringung"). Das Einbringen in die Wunde kann auch durch ein abstoßend wirkendes Magnetfeld forciert werden ("aktive Einbringung"), das entweder durch einen Dauer- oder einen Elektromagneten ausgeübt wird, oder aber durch ein anziehendes Magnetfeld, das auf der jeweiligen Rückseite des zu behandelnden Körperteils ausgeübt wird.

Die funktionalisierte Oberfläche kann z.B. aus einer Silikatoberfläche bestehen, an welche Proteine, wie z.B. Antikörper oder dergleichen, kovalent gebunden sind. Besagte Beads können bevorzugt Teilchengrößen von 200 nm - 1000 µm aufweisen und aus ferromagnetischen Materialien bestehen, wie z.B. Eisen, Nickel und Kobalt und deren Legierungen.

Die Beads werden wie beschrieben aktiv oder passiv in die Wunde gebracht, wo sie in Kontakt mit dem Exsudat treten, eine definierte Dauer verbleiben und die gewünschten Targets binden. Danach werden sie mit Hilfe eines Dauer- oder eines Elektromagneten aufkonzentriert und aus der Wund entfernt. Hierzu kann z.B. ein Wundverband auf die Wunde gegeben werden, der der Wunde zugewandt ein absorbierendes Material aufweist, hinter welchem ein Dauermagnet angeordnet ist. Die Beads werden zusammen mit den daran gebundenen Targets von dem Magneten angezogen und gelangen in das absorbierende Material, wo sie verbleiben, und zusammen mit der Wundauflage entfernt werden können.

Ebenso kann z.B. eine Schaumauflage, eine Alginatlage, ein folien- oder vliesartiges Wunddistanzgitter, eine CMC-Matte, eine Aktivkohlematte, eine Hydrofasermatte, eine Hydrokolloidplatte, eine gelartige Matte, oder auch Watte auf die Wunde aufgebracht werden, und anschließend der Patient in den Wirkungsbereich eines Elektro- oder Dauermagneten gebracht werden. Dabei wirkt das besagte Produkt als Absorbens für die magnetischen Partikel und die daran gebundenen Targets. Dabei kann vorgesehen sein, dass das besagte Produkt auch Metallionen enthält. Es sind weitere Ausgestaltungen dieses erfinderischen Prinzips möglich, die sich dem Fachmann, der bereits anhand dieser Schilderungen eine ausreichende technische Lehre erhält, ohne weiteres Zutun eigener erfinderischer Tätigkeit erschließen.

In Bezug auf einige desinfizierend wirkende Wirkstoffe bzw. Wirkstoffkomplexe kann außerdem bevorzugt sein, dass bei Verwendung der erfindungsgemäßen Zusammensetzung mit einer Wundauflage diese so gestaltet sind, dass sie in der Wundauflage verbleiben und nicht in die Wunde migrieren. Dies kann insbesondere sinnvoll sein, um ein Keimwachstum in der ggf. steriliserten Wundauflage zu verhindern, das ansonsten durch u.U. vorgesehene nutritive Bestandteile wie z.B. Glucose und Aminosäuren begünstigt würde.

Hier ist einerseits an die bereits beschriebenen Silberdonatoren gedacht, andererseits auch an Quaternäre Ammoniumverbindungen (QAV) wie z.B. Benzalkoniumchlorid, CetylTrimethylammoniumbromid, Cetyl-Pyridiniumchlorid oder Benzethoniumchlorid, oder kationische Tenside wie Distearyldimethylammonium-Chlorid oder Esterquats.

Hierbei handelt es sich um organische Ammoniumverbindungen, bei denen alle vier Valenzen eines Stickstoff-Atoms organisch gebunden sind. QAV reichen sich in Zellmembranen lebender Organismen an und können so die Funktion der Zellmembran beeinträchtigen. Dank dieser Wirkung können insbesondere die kationischen Tenside auch als Desinfektionsmittel eingesetzt werden. Da sie u.U. hautirritierende Eigenschaften aufweisen, ist daher vorgesehen, dass sie in der Wundauflage verbleiben, und dort insbesondere für die Hemmung des Bakterienwachstums in der Wundauflage Verwendung finden. Die Immobiliserung kann beorzugt durch kovalente Bindung der QAV an das Material der Wundauflage erfolgen.

In einer anderen, ebenfalls bevorzugten Ausgestaltung der Erfindung ist vorgesehen, dass es sich bei einem der nutritiv wirkenden Wirkstoffkomplexe um eine Zusammensetzung enthaltend mindestens die Bestandteile eines enteralen und/oder parenteralen Diätetikums handelt. Dabei sind die enthaltenen Bestandteile Beispiele für die zuoberst definierten "nutritiven Wirkstoffe".

Ein parenterales Diätetikum enthält in der Regel Elektrolyte, d.h. Mineralsalze, Kohlenhydrate (meist in Form von Glucose), Aminosäuren, Fette und Fettähnliche Stoffe (Lipide) sowie Vitamine und Spurenelementen und eignet sich für die langfristige Ausschließliche Ernährung eines Patienten. In der Regel liegen diese Bestandteile in niedermolekularer Form vor, was insbesondere bei Applikation in Wunden deren Aufnahme durch das Wundgewebe erleichtert. Nicht enthalten sind insbesondere Proteine, die sowohl bei der parenteralen Infusion als auch bei der Applikation in Wunden u.U. Komplikationen, wie z.B. allergische Reaktionen auslösen können.

Ein enterales Diätetikum ist in der Regel komplexer aufgebaut als ein parenterales Diätetikum, d.h. es enthält höhermolekulare Bestandteile, insbesondere Proteine.

Die Zusammensetzung kann dabei z.B. der eines voll bilanzierten Diätetikums entsprechen, aber auch der eines Diätetikums, bei welchem bestimmte Nährstoffgruppen, insbesondere z.B. Kohlenhydrate oder Ballaststoffe bei unveränderter Beibehaltung der übrigen Inhaltsstoffe entfernt sind, oder eines Diätetikums, das um weitere Wirkstoffe ergänzt ist. Siehe hierzu die Beispiele 1 - 3.

So können z.B., um das Bakterienwachstum zu hemmen, Glucose und andere Kohlenhydratquellen weggelassen sein. Denselben Zweck können bakteriostatische Wirkstoffe oder Wirkstoffkomplexe aufweisen, wie sie weiter unten beschrieben sind.

Weiterhin kann die Zusammensetzung zumindest in Teilen auch der des sogenannten Brottrunks bzw. eines Brottrunk-Trockenextrakts entsprechen. Hierbei handelt es sich Um ein durch durch Milchsäuregärung aus einem Vollkornsauerteigbrot gewonnes Getränk und ist reich an nutritiven Wirkstoffen bzw. Wirkstoffkomplexen, insbesondere Zink, Eisen oder Magnesium, sowie den Vitaminen A, B1, B2, B6, B12, C, D, E, Biotin, Niacin, Folsäure und Pantothensäure. Die bei der Herstellung zum Einsatz kommenden Milchsäurebakterien gehören zur Art *Lactobacillus reuteri* und sind aufgrund der fehlenden Pasteurisierung noch vital. Durch seinen niedrigen pH-Wert sowie die lebenden Milchsäurebakterien ist dieses Substrat in der Lage, Matrixproteasen zu inhibieren (siehe unten). Besagter Trockenextrakt kann z.B. durch Gefriertrocknen hergestellt sein.

Das genannte Diätetikum kann als die oder zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein.

Ebenso kann vorgesehen sein, dass es sich bei einem der nutritiv wirkenden Wirkstoffe bzw. Wirkstoffkomplexe um Insulin, rekombinantes Insulin, Proinsulin, einen insulinähnlichen

Wachstumsfaktor (Insulin-like growth factor, IGF), ein Insulinmimetikum und/oder einen diabetikerspezifischen, nicht glucose- bzw. saccharosebasierenden Energieträger handelt.

Auch diese Stoffe stellen eine topische nutritive Versorgung einer Wunde sicher und fallen damit unter die obige Definition. So führt Insulin topisch angewandt zu einer vermehrten Glucoseaufnahme durch die Zellen der Wundregion. Dasselbe gilt für Proinsulin, insulinähnliche Wachstumsfaktoren oder Insulinmimetika, also Moleküle, die im Organismus insulinähnliche Wirkungen hervorrufen. Diese Art der topischen Wundversorgung kann insbesondere bei Diabetikern angezeigt sein. Hier ist die zelluläre, insulinvermittelte Glucoseaufnahme durch wenigstens temporären Mangel an Insulin erheblich gestört. In diesem Zusammenhang ist insbesondere wichtig, dass gerade Diabetiker häufig und chronischen ödematösen Wunden leiden, wie Ulcus cruris und dergleichen. Eine topische Insulintherapie kann hier die Wundheilung erheblich fördern.

In diesem Zusammenhang können jedoch auch diabetikerspezifische, nicht glucose- bzw. saccharosebasierenden Energieträger vorteilhaft sein. Hierunter fallen im Folgenden insbesondere Fructose, Galactose und andere nicht-glucose- bzw. nicht-saccharosebasierende Energieträger (Fettsäuren etc.). Der Fachmann kann der einschlägigen Literatur ohne weiteres erfinderisches Zutun auch andere diabetikerspezifische nicht glucose- bzw. saccharosebasierende Energieträger entnehmen.

Weiterhin ist bevorzugt vorgesehen" dass es sich bei einem der Proteasen hemmend wirkenden Wirkstoffe und/oder Wirkstoffkomplexe um Proteasehemmer, superabsorbierende Polymere, Chelatoren für zweiwertige Kationen, beschichtete magnetische Partikel, Kollagen und/oder einen ansäuernd wirkenden Wirkstoff und/oder Wirkstoffkomplex handelt.

Dabei ist bevorzugt vorgesehen, dass es sich bei einem der ansäuernd wirkenden Wirkstoffe und/oder Wirkstoffkomplexe um ein Wirkelement ausgewählt aus der Gruppe enthaltend Säuren, Puffer, nicht pathogene säureproduzierende Mikroorganismen, Probiotika und/oder Symbiotika handelt.

Chronische Wunden weisen häufig ins alkalische verschobene pH-Wert auf, d.h. einen pH-Wert, der vom normalen pH-Wert der Haut (pH 5.5) in Richtung höherer Werte abweicht. Die genannten ansäurenden Wirkelemente haben auf die Wunde eine ansäuernde Wirkung, d.h. sie sind imstande, den pH-Wert der Wunde zu senken. Dies kann sinnvoll sein, um die wie oben erwähnt in bestimmten Phasen schädlich wirkenden Proteasen, deren Aktivitätsmaximum in der Regel im neutralen bis alkalischen Bereich liegt (d.h. optimiert ist an den pH-Wert chronischer Wunden), zu hemmen.

Ziel dabei ist, den pH-Wert der Wunde auf einen Wert unterhalb von pH 7, bevorzugt unterhalb von pH 6, und besonders bevorzugt unterhalb von pH 5.5 einzustellen. Da es sich bei dem pH-Wert bekanntermaßen um ein Maß für die Protonenkonzentration handelt, erhält der Fachmann bereits anhand dieser Werte eine ausreichende technische Lehre, die ihn befähigt, erfindungsgemäße Zusammensetzungen ohne weiteres Zutun eigener erfinderischer Tätigkeit herzustellen.

Ein weiterer Faktor liegt darin, dass viele infektiöse Pilze im neutralen bis alkalischen Bereich wachsen und im sauren Bereich gehemmt werden. Die extrinsische Ansäuerung von außen trägt also auch zu einer mykostatischen Wirkung der Zusammensetzung bei.

Bei den erwähnten Säuren handelt es sich dabei bevorzugt um organische Säuren. Hier ist bevorzugt an Ameisensäure, Essigsäure, Fruchtsäuren wie Citronensäure, Apfelsäure und Weinsäure, Milchsäure, Gluconsäure, α-Hydroxycaprylsäure, Fumarsäure oder Bernsteinsäure gedacht. Der Fachmann kann der einschlägigen Literatur ohne weiteres erfinderisches Zutun auch andere geeignete Säuren entnehmen. Ein Teil dieser Säuren kann in kristalliner Form verfügbar gemacht werden. Ein anderer Teil dieser Säuren liegt in flüssiger Form vor.

Bei dem Puffer handelt es sich bevorzugt um einen im sauren wirkenden Puffer. Dabei kann es sich z.B. um einen Essigsäure/Acetat-Puffer oder einen Aluminium-Eisen-Puffer handeln. Besonders bevorzugte Puffersysteme sind die im Blut von Säugetieren anzutreffenden Puffersysteme, nämlich das Kohlensäure-Hydrogencarbonat-Puffersystem (H₂CO₃ + H₂O ↔ H₃O⁺ + HCO₃⁻), Phosphatpuffer (H₂PO₄⁻ + H₂O ↔ H₃O⁺ + HPO₄²⁻), Proteinatpuffer (Pufferwirkung durch amphotere (Plasma-)Proteine oder ggf. auch Hämoglobin (Hb·H⁺ + H₂O ↔ H₃O⁺ + Hb).

Der Fachmann kann der einschlägigen Literatur ohne weiteres erfinderisches Zutun auch andere geeignete Puffer entnehmen.

Gemeinsam ist den genannten Säuren und Puffern, dass sie als die oder zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein können.

Bei den nicht pathogenen, säureproduzierende Bakterien handelt es sich bevorzugt um Milchsäurebakterien. Dabei ist insbesondere an Bifidobakterien, Laktokokken, Laktobazillen, insbesondere an die Arten *Lactobacillus rhamnosus, Lactobacillus acidophilus, Bifidobacterium infantis, Bifidobacterium longum, Bifidobacterium breve, Bifidobacterium lactis, Lactococcus lactis, Streptococcus thermophilus, Lactobacillus johnsonii, Lactobacillus delbrueckii, Lactobacillus reuter Bifidobacterium animalis, Lactobacillus plantarum, Lactobacillus casei, Lactobacillus salivarius, Lactobacillus bulgaricus, Lactobacillus acidophilus DDS-1, Lactobacillus paracasei, und*/*oder Lactobacillus sporogenes* gedacht.

All diesen Bakterien ist gemein, dass sie Zucker und andere Substrate (insbesondere Lactose und Präbiotika, siehe unten) anaerob verstoffwechseln und dabei Milchsäure (Lactat) produzieren. Sie können dabei im umliegenden Milieu einen pH-Wert von 4,5 - 4,0 erzeugen und so dazu beitragen, die genannten Proteasen zu hemmen. Hinzu kommt, dass die nicht pathogenen (insbesondere nicht Entzündungen erregenden) Milchsäurebakterien dazu beitragen können, dass pathogene Mikroorganismen in der Wunde kompetitiv gehemmt werden können, z.B. als Nahrungskonkurrenten, durch Abscheiden von Hemmstoffen oder durch Schaffung eines für die genannten pathogenen Mikroorganismen nicht geeigneten sauren Milieus. Der Fachmann kann der einschlägigen Literatur ohne weiteres erfinderisches Zutun auch andere geeignete nicht pathogene, säureproduzierende Bakterien entnehmen.

In diesem Zusammenhang kann auch vorgesehen sein, in der Zusammensetzung rekombinante Mikroorganismen einzusetzen, denen auf rekombinatem Wege die Eigenschaft verliehen wurde, Hemmstoffe gegen pathogene Mikroorganismen zu bilden und freizusetzen.

Diese nicht pathogenen, säureproduzierende Bakterien können z.B. in getrockneter Form vorliegen (ähnlich den käuflich erhältlichen getrockneten Joghurtkulturen), dergestalt, dass Sie bei Kontakt mit Feuchtigkeit, insbesondere Exsudat, aktivierbar sind. Die genannten Milchsäurebakterien werden häufig auch als Probiotika bezeichnet.

Bei den genannten Präbiotika handelt es sich um Stoffe, die eine selektive Nahrungsgrundlage für die genannten nicht pathogenen, säureproduzierende Bakterien darstellen und damit deren Vermehrung zuungunsten ggf. vorhandener pathogener keime begünstigen. Sie sind z.B. in Chicorée, Schwarzwurzeln, Topinambur und vielen anderen un- oder wenig verarbeiteten pflanzlichen Lebensmitteln sind Präbiotika enthalten. Beispiele für Präbiotika sind z.B. Fructooligosaccharide, Inulin, Transgalactooligosaccharide, Xylooligosaccharide, Mannooligosaccharide, Topinambursaft-Pulver oder Lactulose. Der Fachmann kann der einschlägigen Literatur ohne weiteres erfinderisches Zutun auch andere geeignete Präbiotika entnehmen. Diese Probiotika können bereits allein für sich eine ansäuernde Wirkung aufweisen, nämlich dann, wenn in der Wunde bereits vorhandene nicht pathogene, säureproduzierende Bakterien durch deren Anwesenheit in ihrem Wachstum gefördert werden und ein saures Milieu erzeugen.

Bei Symbiotika handelt es sich um einen Oberbegriff für Gemische aus Probiotika und Präbiotika.

Sowohl die Mikroorganismen, die ggf. in getrockneter Form vorliegen, als auch die Prä- sowie die Symbiotika können als die oder zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein.

Die erwähnten Proteasehemmer wirken hemmend auf Proteasen in der Wundregion, die im Laufe der Wundheilung schädlich werden können, insbesondere im pathologischen Exsudat (siehe oben). Dies sind insbesondere Matrix-Metalloproteasen (MMP), deren katalytische Aktivität z.T. von einem zugeordneten Metallion (z.B. Ca²⁺, Zn²⁺) abhängt. Bekannt sind hier insbesondere die Matrix-Metalloproteasen MMP-1 - MMP-10. Dabei ist bevorzugt ist an die Verwendung von sogenannten TIMPs ("tissue inhibitors of matrix metalloproteinases") gedacht. Hierbei handelt es sich um Proteine, die die Aktivität der MMPs durch spezifische Bindung an deren katalytische Zentren binden. Dadurch kann der in der Regenerationsphase der Wunde schädliche Ab- und Umbau des Gewebes durch MMPs verhindert werden. Als Kandidaten kommen hier insbesondere TIMP-1, TIMP-2, TIMP-2 und TIMP-4 in Frage.

Die erwähnten Chelatoren können zweiwertige Metallionen binden, damit die Medienkonzentration der besagten Kationen reduzieren und so die Aktivität der Metalloproteasen reduzieren. Hierbei handelt es sich beispielsweise um EGTA oder EDTA.

Weiterhin können auch superabsorbierende Polymere neben Wasser auch Proteine und zweiwertige Kationen binden. Die Bindung von Proteinen schließt dabei, wie experimentelle Untersuchungen ergeben haben, auch die Bindung von Matrixproteasen ein. Diese werden also aus dem Exsudat entnommen und gebunden und somit der Wundregion entzogen. Die Bindung von zweiwertigen Kationen trägt außerdem zur Deaktivierung der Matrixproteasen bei. Aus diesem Grunde können auch superabsorbierende Polymere als Zusammensetzung enthaltend mindestens einen Proteasen hemmend wirkenden Wirkstoffe und/oder Wirkstoffkomplex verstanden werden.

Die genannten Eigenschaften sind grundsätzlich bekannt Auch existieren Berichte, dass mit Ringerlösung benetzte, superabsorbierende Polymere enthaltende Wundauflagen Metalloproteasen inaktivieren können, und zwar durch direkte Bindung oder durch Bindung von zweiwertigen Kationen (Smola et al, "Polyacrylate-superabsorber inhibits excessive metalloprotease activity in wound fluid from non-healing wounds", abstracts of the ETRS annual Meeting, Pisa, 2007).

Bei den mit Ringerlösung benetzten, superabsorbierende Polymere enthaltenden Wundauflagen handelt es sich um Spülkörper, die auf eine Wunde aufgelegt werden, in einem zyklischen Prozess Ringerlösung an die Wunde abgeben und wieder aufnehmen und dabei die Wunde spülen. Während des Spülprozesses werden auch Matrixproteasen und zweiwertige Kationen aus der Wunde ausgewaschen und in die Wundauflage verbracht, wo sie durch die dort befindlichen superabsorbierenden Polymere gebunden werden. Aufgrund des passiven Spülprinzips ist dies jedoch ein sehr langwieriger Vorgang, der mitunter eine längere Zeit benötigt als die mittlere Verweildauer der Wundauflage auf der Wunde beträgt.

Hingegen führt die hier durch die Erfinder erstmals vorgeschlagene Verwendung von superabsorbierenden Polymeren in einer im Wesentlichen trockenen, absorbierenden Wundauflage zu einer Nettoaufnahme an Flüssigkeit, insbesondere an Exsudat, und damit zu einer sehr viel schnelleren Bindung von Matrixproteasen und zweiwertigen Kationen.

Weiterhin kann als Zusammensetzung enthaltend mindestens einen Proteasen hemmend wirkenden Wirkstoffe und/oder Wirkstoffkomplexe auch ein Gemisch enthaltend magnetische Partikel ("beads") verstanden werden, die mit einer funktionalisierten Oberfläche versehen sind. Besagte funktionalisierte Oberfläche kann z.B. Antikörper gegen eine oder mehrere Matrixproteasen aufweisen. Ebenso kann besagte Oberfläche ein Substrat für Matrixproteasen, wie z.B. Kollagen (insbesondere Kollagen Typ IV) aufweisen, an welche die Proteasen über ihre Substratbindungsdomäne bereitwillig binden.

Das Wirkprinzip sowie etwaige stoffliche Ausgestaltungen sind bereits oben beschrieben. Die Beads werden passiv oder aktiv in die Wunde gegeben, wo sie in Kontakt mit dem Exsudat treten, dort eine Weile verbleiben und die gewünschten Matrixproteasen binden. Danach werden sie mit Hilfe eines Dauer- oder eines Elektromagneten aufkonzentriert und aus der Wunde entfernt. Hierzu kann z.B. ein Wundverband auf die Wunde gegeben werden, der der Wunde zugewandt ein absorbierendes Material aufweist, hinter welchem ein Dauermagnet angeordnet ist. Die Beads werden zusammen mit den daran gebundenen Targets von dem Magneten angezogen und gelangen in das absorbierende Material, wo sie verbleiben, und zusammen mit der Wundauflage entfernt werden können. Eine Unterdrucktherapie kann sich im Zusammenhang mit der bereits erwähnten Anwendung von magnetischen Partikeln als vorteilhaft erweisen.

Erstmals werden mit dem geschilderten Ansatz die Mechanismen von Magnetismus, spezifischer enzymatische Aktivität sowie enzymkinetisch relevanter Mechanismen der Hemmung miteinander in das Segment der Wundbehandlung gebracht, und in der Summe lassen sich Manipulationen an den Prozessen der Wundheilung in einer Gewebetiefe erreichen, die sonst nur über chirurgische Schritte denkbar sind. Der Einschnitt in gesundes Gewebe sowie die Applikation über systemische Wege scheinen vergleichsweise ungünstig, auch wenn unbekannt ist, wie tief dieser Einfluss wirkt.

Die Bildung der Summe über eine schellenförmige, fußfesselartige Ringkonstruktion mit gegensätzlicher, gleicher oder temporär nachgeschalteter Polarisierung ist denkbar.

Ziel ist es, bestehende interstitielle Wege mit magnetischer Triebkraft zu nutzen, um den unterhalb der Wundoberfläche gelegenen Regionen Stoffe zuzuführen, die über kontrollierte Eigenschaften mit erwünschten Einflüssen auf die Wundheilung verfügen. So kommt es zu einem gerichteten Transport von Stoffen in die Gewebetiefe und wieder heraus. Das Anbieten von überschüssigem Substrat in Form von Kollagen, an das Proteasen binden, um sodann später magnetisch extrahiert zu werden, bildet eine Möglichkeit. Auch können hygroskopisch wirksame Stoffe, Tenside, Nährstoffe, Pharmaka aller Klassen und Stoffgruppen (=zur Behandlung von Erkrankungen geeigneter Arzneikomplex) sowie andere Wirkstoffe und Komplexe eingegeben werden, die stets an magnetische Partikel gebunden sind.

Die zuzugebenden Stoffe können vor über verschiedene Gemische der Wundoberfläche zugeordnet werden, beispielsweise über Aerosole, Flüssigkeiten oder Granulate.

Diese Verbindungen können über Freisetzungen von Stoffen, wie z.B. Arzneimittel, in der Gewebetiefe verfügen, so dass nicht nur oberflächenaktive, sondern auch pharmazeutisch wirksame Prozesse, bildgebende Verfahren über freigesetzte Kontrastmittel, antibiotische Maßnahmen, zytostatische Schritte, Immunmodulationen, Ansäuerungen der Wundregion oder Komplexbildungen angestoßen werden.

Auch die Verwendung von Kollagen als solches kann bereits eine Proteasen hemmende Wirkung aufweisen, die auf dem Prinzip der kompetitiven Hemmnung beruht. Diesem Prinzip liegt zugrunde, dass den Proteasen ein Substrat, nämlich Kollagen, im Überangebot zur Verfügung gestellt wird, das diese bevorzugt angreifen. Damit üben sie ihre Wirkung nicht oder zumindest nur reduziert in der Wundregion aus. Die Verwendung von Kollagen kann jedoch noch weitere Vorteile aufweisen, auf die weiter unten eingegangen wird.

Überdies kann die Zusammensetzung auch analgetische, d.h. schmerzstillende Wirkstoffe enthalten. Hier kommen prinzipiell alle jene Wirkstoffe in Frage, die in Hauptgruppe 05 der sog. "Roten Liste" aufgeführt sind. Besonders bevorzugt sind dabei insbesondere entzündungshemmende Wirkstoffe wie z.B. sogenannte Cox-Hemmer oder NSAID (non steroidal anti-inflammatroy drugs), so z.B. Propionsäurederivate wie Naproxen, Ibuprofen, Ketoprofen, Fenoprofen, Flurbiprofen, Dexibuprofen oder Tiaprofensäure, Essigsäurederivate wie z.B. Diclofenac, Alclofenac, Fenclofenac, Etodolac, Aceclofenac, Sulindac oder Indometacin, Pyrrolessigsäuren wie Ketorolac oder Tolmetin, N-Phenylessigsäuren wie Mefenaminsäure oder Flufenaminsäure, Salicylate wie Acetylsalicylsäure, Salicylsäure oder Diffunisal, Pyrazolonderivate wie Phenylbutazon, Oxicamderivate wie Piroxicam, Tenooxicam, Meloxicam oder Lornoxicam, Enolsäurederivate wie Aminopyren oder Antipyren, Phenole wie Acetaminophen und dergleichen. Hinzu kommen COX-2-Inhibitoren wie beispielsweise Rofecoxib, Lumiracoxib oder Celecoxib.

Überdies kann es sich bei den schmerzstillenden Wirkstoffen auch um nicht entzündungshemmende Wirkstoffe handeln, so z.B. um Opiate, lokale Anaesthetika wie Lidocain, Mepivacain, Prilocain, Procain, Syntocain, Tetracain, Gingicain, Articain, Bupivacain, Butanilicain, Chloroprocain, oder z.B. Polidocanol.

Weiterhin kann die Zusammensetzung auch entzündungshemmende Wirkstoffe aufweisen, die ggf. sekundär einen analgetischen Effekt aufweisen, so z.B. - neben den oben genannten, z.T. ebenfalls entzündungshemmend wirkenden Analgetika - Hormone, insbesondere Cortison und Cortikoide, insbesondere Glucokortikoide (z. B. Cortison, Cloprednol, Prednison, Prednisolon, Methylprednisolon, Deflazacort, Fluocortolon, Triamcinolon, Dexamethason, Betamethason) und Mineralokortikoide (z.B. Aldosteron, Desoxycorticosteron, Fludrocortison).

Grundsätzlich kann es vorteilhaft sein, die besagten Wirkstoffe bzw. Wirkstoffkomplexe - insbesondere die nutritiven, desinfizierenden und/oder ggf. auch die analgetischen - über den akuten Versorgungsbedarf der Wunde hinaus zu überdosieren, da z. B. Teile der Wirkstoffe in einer Wundauflage verbleiben. Diese Überdosierung dient jedoch nicht dem Ziel, eine allgemeine Nährstoffmangelsitutation eines Patienten zu beheben oder eine systemische Sepsis zu unterbinden, da eine systemische Wirkung wie oben erwähnt weder angestrebt noch erwünscht ist. Gleichwohl kann, wie im Folgenden ausgeführt wird, die gewählte Dosierung sogar über der Dosierung liegen, die z.B. als Tagesdosierung bei oraler oder enteraler Einnahme empfohlen wird. Dies ist insbesondere deswegen sinnvoll, weil z.B. bei Verwendung der Zusammensetzung in einer Wundauflage letztere häufig über längere Zeit auf der Wunde verbleiben soll.

Falls z.B. Zusammensetzungen verwendet werden, die denjenigen eines Diätetikums entsprechen, ist bevorzugt vorgesehen, dass die verwendeten Mengen z.B. pro Wundauflageprodukt (also z.B. Schaumstoffmatte oder Wundauflage enthaltend SAP) im Bereich zwischen 10 und 200 % der von der DGE (Deutsche Gesellschaft für Ernährungsmedizin) empfohlenen Tagesdosierungen liegen. Besonders bevorzugt liegen sie im Bereich zwischen 30 und 100 % der empfohlenen Tagesdosierungen.

Dabei können die genannten Zusammensetzungen z.B. in gelöster Form in die Wundauflage eingebracht werden bzw. schon vorkonfektioniert in die Wundauflage eingebracht sein. Alternativ können die Zusammensetzung in getrockneter Form vorkonfektioniert in die Wundauflage eingebracht sein.

Falls z. B. Zusammensetzungen verwendet werden, die desinfizierende Wirkstoffe enthalten, so ist bevorzugt vorgesehen, dass die verwendeten Mengen z. B. pro Wundauflageprodukt der 10-fachen empfohlenen Tagesdosis pro kg Körpergewicht entsprechen.

In weiteren bevorzugten Ausgestaltungen enthält die Zusammensetzung überdies einen oder mehrere Wirkstoffe, die ausgewählt sind aus der Gruppe enthaltend orthomolekulare Nährstoffe, Nutraceuticals, Phytochemicals, Antioxidantien, Wachstumsfaktoren, Erdölbasierende Zugpräparate, Methyxanthine, Gerbstoffe, Tacrolimus, Pinecrolimus, ATP, Harnstoff, Sympathomimetika, Parasympatholytika, Aktivkohle, Octenidin, Polihexanid, Homöopathika, Q10, Dickungsmittel, Karaja, Pektin, Agar, Aloe vera, Hämostyptika, Tierspeichel wie Maden- oder Hundespeichel, Spinnwebenproteine, Kollagen, Hygroskopika, Glycerin, Biofilm beschädigende Substanzen, Triacetin, Zinkoxid, lichtabsorbierende Bestandteile, geruchshemmende Stoffe, Gelbildner, exsudationsfördernde Stoffe, abschwellend wirkende Stoffe, Radikalfänger, und/oder Honig bzw. dessen Inhaltsstoffe. Die meisten dieser Bestandteile fallen unter die obigen Definition der nutritiven, desinfizierenden und/oder Proteasen hemmenden wirkenden Wirkstoffe bzw. Wirkstoffkomplexe.

Orthomolekulare Nährstoffe sind im Rahmen des Konzepts der orthomolekularen Medizin, einer maßgeblich von Linus Pauling beeinflussten alternativmedizinischen Methode, verwendete Nährstoffe, insbesondere Vitamine und Mineralstoffe, die in vorgesehenen z.T. hochdosierten Dosierungsschemata verabreicht werden.

Nutraceuticals sind Nahrungsmittel, die mit zusätzlichen Inhaltsstoffen (Nahrungsergänzungsmittel) angereichert werden, die einen positiven Effekt auf die Gesundheit haben sollen. Zugesetzt werden vor allem Vitamine, Mineralstoffe, Bakterienkulturen und ungesättigte Fettsäuren

Phytochemicals sind insbesondere sekundäre Pflanzenstoffe; zu ihnen zählen u.a. Carotinoide, Polyphenole und Sterole. Ihnen wird unter anderem eine antioxidative Wirkung und die Bekämpfung freier Radikale zugeschrieben, die Förderung der Immunabwehr und die Senkung des Cholesterinspiegels.

Als Antioxidantien werden Wirkstoffe bezeichnet, die die Oxidation empfindlicher Moleküle zu verhindern, insbesondere von DNA und Proteinen. In der Regel wirken sie als Radikalfänger. Nach Art des chemischen Wirkmechanismus können Antioxidantien in "Antioxidantien", "reduzierende Substanzen" und "Antioxidantien mit synergistischen Effekten" typisiert werden. Definition für die sogenannten richtigen Antioxidantien ist der Wirkmechanismus, der darin besteht, dass Kettenreaktionen durch das Einfangen von freien Radikalen blockiert werden. Beispiele solcher Antioxidantien sind BHA und BHT. Im Gegensatz dazu wirkt beispielsweise Ascorbinsäure als reduzierende Substanz dadurch, dass sie sich leichter oxidieren läßt als die zu schützenden Moleküle und somit einen Schutz darstellt. Zur letztgenannten Gruppe der synergistischen Antioxidantien zählt beispielsweise Natrium-EDTA, welches die antioxidative Wirkung verstärkt, indem sie Metallionen komplexiert.

Im Rahmen der vorliegenden Erfindung ist insbesondere an folgende Antioxidatien gedacht: Antioxidantien, die zur Vitamin-E-Familie gehören, Carotinoide, insbesondere Lycopin und β-Carotin, Glutathion, Transferrin, Albumin, Coeruloplasmin, Hämopexin, Haptoglobin, antioxidative Enzyme, insbesondere Superoxiddismutase (SOD), Glutathionperoxidase (GPX) und Katalase, Zinnchlorid, Ascorbinsäure (Vitamin C) und deren Derivate Natrium-L-Ascorbat, Calcium-L-Ascorbat, Isoascorbinsäure, Natriumisoascorbat und Ascorbylpalmitat, Butylhydroxyanisol, Butylhydroxytoluol, Calcium-Dinatrium-EDTA, Gallat, insbesondere Propylgallat, Octylgallat und Dodecylgallat (Laurylgallat), Lecithin, Milchsäure, Mehrfach-Phosphate wie Diphosphat, Triphosphat und Polyphosphat, Schwefeldioxid, Natriumsulfit, Natriumbisulfit, Natriumdisulfit, Kaliumsulfit, Calciumsulfit, Calciumhydrogensulfit, Kaliumbisulfit, Selen, Tocopherol (Vitamin E), Alpha-Tocopherol, Gamma-Tocopherol, Delta-Tocopherol, Zinn-II-Chlorid, Zitronensäure sowie Natriumcitrat und Kaliumcitrat, reduzierende Agenzien wie Acetylcystein.

Als Wachstumsfaktoren werden wachstumsrelevante Polypeptide bzw. Proteine bezeichnet, die als Signale von einer Zelle auf eine zweite übertragen werden und damit wachstumsrelevante Informationen weiterleiten. Sie spielen insbesondere eine Rolle bei der Entwicklung von mehrzelligen Organismen. Die wichtigsten Wachstumsfaktoren sind:
● Transforming growth factor *Beta* (TGF-B)
● Granulocyte-colony stimulating factor (G-CSF)
● Granulocyte-macrophage colony stimulating factor (GM-CSF)
● Nerve growth factor (NGF)
● Neurotrophins
● Platelet-derived growth factor (PDGF)
● Erythropoietin (EPO)
● Thrombopoietin (TPO)
● Myostatin (GDF-8)
● Growth differentiation factor-9 (GDF9)
● Basic fibroblast growth factor (bFGF or FGF2)
● Vascular endothelial growth factor (VEGF)
● Platelet derived growth factor (PDGF)
● Epidermal growth factor (EGF)
● Hepatocyte growth factor (HGF)

Erdölbasierende Zugpräparate weisen eine exsudationsfördernde sowie nekrolytische Wirkung auf. Sie können daher insbesondere dekontaminierend wirken. Unter dem genannten Begriff versteht man insbesondere die sogenannte schwarze Zugsalbe sowie auf Ichtyol basierende Präparate, aber auch Schieferölsulfonate. Schieferölsulfonate wie z.B. Ammoniumbituminosulfat sind durch Sulfonierung wasserlöslich. Die genannten Präparate können erfindungsgemäß in fettige oder wässrige Bestandteile der Umhüllung einer Wundauflage eingearbeitet sein und als erste Wundkontaktschicht aufliegen.

Methyxanthine sind eine Gruppe von Alkaloiden, die für gewöhnlich als milde Stimulantien eingesetzt werden sowie zur Behandlung von Asthma bronchiale. Sie umfassen Koffein, Theophyllin und Theobromin. Xanthine sind Purin-Derivate. Sie wirken konstringierend und eher abschwellend, so dass in der Wundregion ggf. vorhandene Ödeme reduziert werden und nutritive, desinfizierende und/oder Proteasen hemmende Wirkstoffe nicht unnötig verdünnt werden.

Gerbstoffe wirken adstringierend, d.h. ödemreduzierend, entzündungshemmend, antibakteriell, antiviral und neutralisieren Gifte.

Tacrolimus (auch FK506 oder FK-506) ist ein Makrolid aus dem Bakterium *Streptomyces tsukubaensis.* Tacrolimus wird u.A. als selektives Immunsuppressivum gegen Abstoßungsreaktionen bei der Organtransplantation verwendet Tacrolimus wirkt immunsuppressiv und antimikrobiell. Es ist in der Wirkung mit dem Polypeptid Ciclosporin vergleichbar, kann aber in niedrigeren Dosen eingesetzt werden. Tacrolimus greift in den Stoffwechsel von T-Zellen ein und hemmt deren Aktivität. Es bindet an den zytosolischen Rezeptor, einem sogenannten Immunophilin innerhalb der Zielzelle. Der Komplex aus Immunophilin und Tacrolimus lagert sich an die Serin-Threonin-Phosphatase Calcineurin. Calcineurin kann nun nicht mehr aktiviert werden. Für die Substanz Tacrolimus gilt im wesentlichen dasselbe.

ATP ist ein Nucleotid, bestehend aus dem Triphosphat des Nucleosids Adenosin, und als solches ein energiereicher Baustein der Nukleinsäuren DNA und RNA. ATP ist jedoch auch die universelle Form unmittelbar verfügbarer Energie in jeder Zelle und gleichzeitig ein wichtiger Regulator energieliefernder Prozesse. ATP kann aus Energiespeichern (Glykogen, Kreatin-Phosphat) bei Bedarf freigesetzt werden. Die Hinzufügung von ATP zu der erfindungsgemäßen Zusammensetzung stellt eine glucosefreie Energiequelle dar und kann insbesondere bei Diabetes angezeigt sein, um den Energiehaushalt der Zellen der Wundregion zu verbessern.

Harnstoff weist eine hohe Wasserbindungsfähigkeit auf und überdies keratolytische Eigenschaften. Weiterhin dient es als Feuchtigkeitsspender zur Bekämpfung von atopischen Ekzemen und Lichenerkrankungen und eignet sich daher besonders bevorzugt zur Verwendung in einer erfindungsgemäßen Zusammensetzung.

Nekrolytika sind Agenzien, die nekrotisches Gewebe aufzulösen vermögen. Hierbei kann es sich z.B. um die hier beschriebenen erdölbasierenden Zugpräparate handeln. Weitere mögliche nekrolytische Agenzien sind z.B. Harnstoff oder Tierspeichel, die beide weiter unten ebenfalls noch beschrieben werden.

Sympathomimetika wirken stimulierend auf den sympathischen Teil des vegetativen Nervensystems. Dadurch wird eine Erhöhung des Blutdruckes und der Herzfrequenz, eine Erweiterung der Atemwege, eine allgemeine Leistungssteigerung und ein erhöhter Energieverbrauch bewirkt. Im Zusammenhang mit der erfindungsgemäßen Zusammensetzung weisen diese Stoffe eine abschwellende Wirkung sowie eine ödemreduzierende Wirkung auf.

Parasympatholytika sind Arzneimittel, welche der Wirkung des Parasympathikus entgegenwirken. Die therapeutische Anwendung der Parasympatholytika wird durch eine mangelnde Organselektivität erschwert. So fördert Atropin, als Medikament zur Therapie einer chronisch-obstuktiven Bronchitis, nicht nur die Bronchodilatation, sondern sorgt auch für eine erhöhte Herzfrequenz, ein Weitstellen der Pupillen und eine Kontraktion der glatten Muskulatur. Es stellen sich bei Verwendung dieser Substanzen vergleichbare Effekte ein wie bei den oben beschriebenen Sympathomimetika.

Aktivkohle ist eine feinkörnige Kohle mit großer innerer Oberfläche, die als Adsorptionsmittel unter anderem in Chemie, Medizin, Wasser- und Abwasserbehandlung sowie Lüftungs- und Klimatechnik eingesetzt wird. In eine erfindungsgemäße Zusammensetzung eingebracht kann sie zur Bindung und damit Befreiung der Wundregion von Giftstoffen aus Stoffwechselprozessen und Keimen beitragen.

Q10 oder Coenzym Q10 ist ein Chinon-Derivat mit lipophiler Isoprenoid-Seitenkette, strukturell verwandt mit Vitamin K und Vitamin E. Coenzym Q10 ist ein essentieller Elektronen- und Protonen-Überträger zwischen dem Komplex I bzw. Komplex II und dem Komplex III der Atmungsketetn und kann im Rahmen einer erfindungsgemäßen Zusammensetzung den Energiestoffwechsel der Zellen im Wundbereich bei Resorption mit Nährstoffen unterstützen.

Dickungsmittel werden - in der Regel wässrigen - Lösungen zugesetzt, um ihre Viskosität zu erhöhen. Sie sind in erster Linie in der Lage, Wasser zu binden. Durch Entzug von ungebundenem Wasser kommt es zur Erhöhung der Viskosität. Ab einer für jedes Verdickungsmittel charakteristischen Konzentration treten zu diesem Effekt auch noch Netzwerkeffekte auf, die zu einer meist überproportionalen Erhöhung der Viskosität führen. Dickungsmittel ermöglichen in Zusammenhang mit der erfindungsgemäßen Zusammensetzung eine Wundbettadaptation und eine Oberflächenmaximierung zur Resorptionsfläche.

Geeignete Dickungsmittel sind z.B. Karaya (Indischer Traganth, Karayagummi, E 416), ein aus Kohlenhydraten und Galacturonsäure bestehender Pflanzengummi (Sekret des indischen Sterculia-Baumes), Alginsäure, Agar, Carrageen, Johannisbrotkernmehl, Guarkernmehl, Traganth, Gummi arabicum,Xanthan, Karaya, Tarakernmehl, Gellan, Pektin, Zellulose, Zelluloseether, Carboxymethylcellulose, Hydroxypropylcellulose, Hydroxypropylmethylcellulose, Methylcellulose, Methylethylcellulose, modifizierte Stärke, Eigelb, Gelatine, Mehlschwitze, Mehlbutter, Sago, und Stärke

Pektine sind pflanzliche Polysaccharide, genauer Polyuronide, die im wesentlichen aus α-1,4-glykosidisch verknüpften D-Galacturonsäure-Einheiten bestehen. Viele Mikroorganismen sind in der Lage, Pektine zu verstoffwechseln. Aufgrund ihrer Fähigkeit, Gele zu bilden, kommen Pektine ebenfalls als Verdickungsmittel im obigen Sinne in Frage. Hinzu kommt die Fähigkeit, als Komplexbildner bei der Entgiftung bei Schwermetallvergiftungen mitzuwirken, und ist daher im Rahmen einer erfindungsgemäßen Zusammensetzung besonders geeignet.

Aloe vera ist eine Pflanzenart aus der Gattung der Aloen die ein gleichnamiges Gel produziert, das auch als Acemannan vbezeichnet wird und ein langkettiges Polysaccharid aufweist. Diese Substanz aktiviert in In-Vitro-Experimenten das Immunsystem, schützt die Zellmembranen und ist antibakteriell, antiviral und antimykotisch wirksam. Diese Substanz wird besonders gut über den Magen-Darm-Trakt in den Körper aufgenommen, kann aber auch im Wunsdbereich verwendet werden. Überdies, t enthält die Aloe vera Mineralstoffe (Calcium, Magnesium, Zink, Selen u.a.), Vitamine, Aminosäuren und sekundäre Pflanzenstoffe (Flavonoide). Vom Begriff "Aloe vera" im Sinne der vorliegenden Erfindung sind daher auch "Aloe vera"-Extrakte erfasst, deren Inhaltsstoffe leichter durch Zellen der Wundregion aufgenommen werden können.

Hämostyptika oder Hämostatika sind Stoffe, die die Blutstillung anregen und so den Wundheilungsprozess ermöglichen, insbesondere Vitamin K, Gerinnungsfaktoren (z.B. Faktor VIII, Faktor IX), trans-4-Aminomethylcyclohexancarbonsäure und weitere.

Unter dem Begriff "Tierspeichel" sollen insbesondere Hundespeichel und Madenspeichel verstanden werden. Hundespeichel weist neben desinfizierenden auch nekrolytische und granulationsfördernde Substanzen und kann auf diese Weise die Heilung von Wunden beschleunigen. Madenspeichel ist der Speichel von in der Wundtherapie verwendeten Insekten, insbesondere Maden (insbesondere der Goldfliegenart *Lucilia sericata*)*.* Besagte Insekten sind imstande, chronische Wunden von nekrotischem Gewebe und Bakterienbefall zu reinigen. Neben der Entfernung von Wundbelägen und Bakterien wird die Wundheilung und das Nachwachsen von frischem Gewebe durch Stoffe, insbesondere Enzyme, die im Speichel der Goldfliegenmaden enthalten sind, angeregt und gefördert.

Bei besagtem Speichel kann insbesondere vorgesehen sein, dass er auf rekombinantem, d.h. gentechnischem Wege hergestellt ist.

Spinnenseide besteht aus langkettigen Proteinmolekülen. Die in den Spinndrüsen produzierten Fäden sind durch die spezielle molekulare Anordnung der beteiligten Aminosäuren sehr dehnbar, extrem belastbar, enorm zugfest und gleichzeitig hochelastisch. Die Spinnfäden sind leicht und wasserfest, besitzen aber dennoch ein hohes Wasseraufnahmevermögen, das dem von Wolle vergleichbar ist. Sie widerstehen mikrobiologischen Angriffen und eignen sich daher besonders zur Verwendung im Zusammenhang mit der erfindungsgemäßen Zusammensetzung, beispielsweise als Hüllmaterial für eine Wundauflage enthaltend die Zusammensetzung oder als partikulärer, desinfizierend wirkender Wirkstoff. Unter den obigen Begriff fällt auch künstliche, insbesondere rekombinant hergestellte Spinnenseide.

Kollagen ist ein bei Menschen und Tieren vorkommendes Strukturprotein des Bindegewebes Im menschlichen Körper ist Kollagen mit über 30 % Anteil am Gesamtgewicht aller Eiweiße (Proteine) das am meisten verbreitete Eiweiß. Kollagen eignet sich im Zusammenhang mit der erfindungsgemäßen Zusammensetzung als Ca²⁺ Fänger zur Reduktion der Aktivität von Proteasen, insbesondere in Zusammenhang mit Vitamin C. Ebenso können sie, wie bereits oben beschrieben, Proteasen hemmen, indem sich durch selbige verdaut werden.

Unter Hygroskopie bezeichnet man in der Chemie und Physik die Eigenschaft eines Stoffes (eines Hygroskopikums), Feuchtigkeit aus der Umgebung (meist in Form von Wasserdampf aus der Luftfeuchtigkeit) zu binden. Hygroskopika können insbesondere auch Exsudate aufnehmen und eigen sich daher besonders zur Verwendung im Zusammenhang mit der erfindungsgemäßen Zusammensetzung. Beispiele sind z.B. superabsorbierende Polymere, Glycerin, Silikate wie Silicagel, Melissinsäure und dergleichen Die besagten Hygroskopika können insbesondere auch eine exsudationsfördernde Wirkung besitzen und daher die Wunddekontamination fördern und begünstigen.

Triacetin (Glycerintriacetat) ist eine Esterverbindung aus Glycerin und Essigsäure. Es wirkt antimikrobiell und wird als Weichmacher und wegen seiner hygroskopischen Wirkung auch als Feuchthaltemittel verwendet.

Biofilme bestehen aus einer dünnen Schleimschicht (Film), in der Mikroorganismen (z.B. Bakterien, Algen, Pilze, Protozoen) eingebettet sind. Biofilme entstehen, wenn Mikroorganismen sich an Grenzflächen ansiedeln. Sie bilden sich überwiegend in wässrigen Systemen, entweder auf der Wasseroberfläche oder auf einer Grenzfläche zu einer festen Phase. In mehr als 60 % aller bakteriellen Infektionskrankheiten schützen sich die Erreger durch die Bildung von Biofilmen vor dem Immunsystem. Hierunter fallen die mikrobielle Kontamination und Besiedlung von Kathetern, Implantaten und Instrumenten.

Biofilme zerstörende Substanzen sind z.B. abrasive Substanzen, wie Kalkmehl, Detergenzien oder schleimlösende Substanzen, insbesondere reduzierend wirkende Agenzien, die Disulfidbrücken auflösen können. Ihre Verwendung im Zusammenhang mit der erfindungsgemäßen Zusammensetzung ermöglicht die Reduktion der Keimbesiedlung und damit eine Verbesserung der Nährstoffzufuhr und/oder eine Verhinderung der Wundinfektion

Weiterhin kann vorgesehen sein, dass die erfindungsgemäße Zusammensetzung abschwellend wirkende Stoffe wie z.B. Extrakte aus Augentrost (*Euphrasia officinalis*)*,* Salbei (*Salvia officinalis*) oder Schlüsselblume (*Primula veris*)*,* Vasokonstriktiva wie Oxymetazolin- oder Xylometazolinhydrochlorid oder Antiödematika. Dies kann hilfreich sein, um Schwellungen im Wundbereich zureduzieren und odematöse Flüssigkeit verfügbar zu machen, so dass sie durch eine absorbierende Wundauflage aufgenommen werden kann.

Weiterhin kann vorgesehen sein, dass die erfindungsgemäße Zusammensetzung lichtabsorbierende Bestandteile aufweist. Diese helfen, den Verlust der strukturellen Integrität bei lichtempfindlichen Bestandteilen (z.B. Zinkoxid, Vitamine) zu vermeiden.

Als lichtabsorbierende Bestandteile kommen insbesondere Pigmente, wie z.B. Titandioxid, in Frage. Diese können in ein Hüllmaterial eingearbeitet sein, aber auch zusammen mit weiteren Bestandteilen z.B. in einer Lösung, einer Emulsion oder Ähnlichem vorliegen. Überdies können Sie auch in Produktverpackungen vorgesehen sein. Diese können jedoch auch ganz allgemein dunkel gehalten sein, um die enthaltenen Inhaltsstoffe vor Licht zu schützen.

Geruchshemmende Stoffe nehmen übelriechende Stoffe auf, binden sie oder verhindern deren Entstehung und verbessern so die Lebensqualität der mit der erfindungsgemäßen Zusammensetzung behandelten Patienten. Hierbei kann es sich z.B. um Aktivkohle, Kräuterextrakte, Parfüms und dergleichen handeln.

Grundsätzlich weisen auch alle genannten desinfizierend wirkenden Wirkstoffe bzw. Wirkstoffkomplexe eine geruchshemmende Wirkung auf.

Weiterhin kann die erfindungsgemäße Zusammensetzung Gelbildner enthalten, wie z.B. Agar, Gelatine, Acrylamid, Agarose oder UV-vernetzbare Gelbildner. Mit deren Hilfe kann ein Gel auf der Wunde ausgebildet sein, das einerseits als Wundabdeckung fungiert und die Feuchthaltung und den Schutz der Wunde sichert, andererseits aber auch als Tröger und Spender der genannten Wirkstoffe bzw. Wirkstoffkomplexe fungiert und eine Zuführung derselben an die Wunde sichert.

Radikalfänger inaktivieren Freie Radikale, die ansonsten biologisches Gewebe unter oxidativen Stress setzen und als Initiator Kettenreaktionen auslösen, die Zell- und Gewebsschäden erzeugen können, insbesondere Veränderungen der zellulären DNA. Hier kommen insbesondere Epigallocatechingallat, Superoxiddismutase, Glutathionperoxidase, Vitamin A, Vitamin C, Vitamin E, Coenzym Q10 und Anthocyane in Frage. Auch Bilirubin, Harnsäure können freie Radikale neutralisieren, ebenso wie das Hormon Melatonin. Radikalfänger sind häufig auch Antioxidantien. Besonders bevorzugt ist dabei insbesondere die Kombination aus Vitamin C und Vitamin E. Eine solche Kombination weist besonders synergistische Effekte in Bezug auf die antioxidative Wirkung auf.

Honig besteht zu 70-80 Gew.-% aus Invertzucker und weist überdies Enzyme, Vitamine, Aminosäuren, Pollen, Aromastoffe und Mineralstoffe. Er hat eine antibakterielle Wirkung und wirkt überdies hygroskopisch und ist aus diesen Gründen besonders vorteilhaft in Verbindung mit den erfindungsgemäßen Zusammensetzungen.

Weiterhin ist bevorzugt vorgesehen, dass es sich bei den Vitaminen um ein oder mehrere Vitamine handelt, die ausgewählt sind aus der Gruppe enthaltend Vitamin B12, Vitamin D, Vitamin C, Vitamin B1, Vitamin B2, Vitamin B6, Niacin und/oder Folsäure.

Bei einem Mangel an Vitamin B12 (Cobalamin) kann es zur Perniziösen Anämie (*Perniziosa*)*,* einer Erkrankung des Blutbildes und zur funikulären Myelose kommen. Die Ursachen für diesen Mangel können zum einen in unzureichender Zufuhr durch Nahrung, wie sie bei veganer Ernährung beobachtet wurde, oder durch unzureichende Resorption verursacht werden. Bei mangelhafter Aufnahmefähigkeit im Magen-Darm-Trakt fehlt dem Organismus im Magensaft der *intrinsic factor,* ein Glykoprotein, das von den Belegzellen des Magens produziert wird und für die Vitamin B12-Aufnahme unabdingbar ist. Der *intrinsic factor* bindet Cobalamin in einem vor Verdauungsenzymen geschützten Komplex und ermöglicht so den Transport in die Darmzellen, von wo aus Vitamin B12 über Bindung an weitere Proteine (Transcobalamine) in die äußeren Gewebe gelangt. Auch eine Störung bei der Aufnahme im terminalen Ileum kann zu einem Mangel führen. Zwar ist ein direkter Zusammenhang zur Wundheilung manchen Quellen unbekannt, jedoch ist Vit. B12 eines der typischen Vitamine mit Substitutionsbedarf des älteren Menschen.

Vitamin D ist eine Sammelbezeichnung für eine Gruppe fettlöslicher Vitamine, die vielfache physiologische Wirkungen haben. Ihr für den Menschen wesentliche Vertreter, das Vitamin D3 (oder Cholecalciferol), ist ein Prohormon, welches der Körper mit Hilfe von UVB-Licht selber in der Haut bilden oder mit der Nahrung zuführen kann.

Vitamin C ist eine organische Säure. Da sie leicht oxidierbar ist, wirkt sie antioxidativ. Ihre wichtigste Eigenschaft ist die physiologische Wirkung als Vitamin, eine Mangel kann sich bei Menschen durch Skorbut manifestieren. Vitamin C ist ein Radikalfänger und hat eine antioxidative Wirkung (es wirkt also als Reduktionsmittel). Es ist ein wichtiger Cofaktor bei der Hydroxylierungsreaktion und ermöglicht damit unter anderem die körpereigene Herstellung von Collagen. Darüber hinaus spielt es eine wichtige Rolle beim Aufbau von Aminosäuren. Durch seine antioxidative Wirkung schützt es andere wichtige Metaboliten und das Erbgut vor der Oxidation bzw. dem Angriff durch freie Radikale, was im Endeffekt einen Schutz der Zelle vor Schäden und somit auch vor Krebs bedeutet. Mit Niacin und Vitamin B6 steuert Vitamin C die Produktion von L-Carnitin, das für die Fettverbrennung in der Muskulatur benötigt wird. Weiterhin begünstigt es die Eisenresorption im Dünndarm.

Thiamin oder Vitamin B1 ist ein wasserlösliches Vitamin aus dem B-Komplex von schwachem, aber charakteristischem Geruch und ist insbesondere für die Funktion des Nervensystems unentbehrlich. Vitamin B1 ist für die Verbrennung von Kohlenhydraten unbedingt erforderlich, wobei es sich als Coenzym selbst verbraucht. Da Gehirn und Nervenzellen auf Energie aus Kohlenhydraten angewiesen sind, wirkt sich ein Thiamin-Mangel besonders auf alle Gehirn- und Nervenfunktionen aus

Vitamin B2 oder Riboflavin dient als Vorstufe für Flavin-Koenzyme (FAD, FMN), die insbesondere in Oxidoreduktasen z. B. im Zitronensäurezyklus eine große Rolle spielen. Dadurch nimmt es im Stoffwechsel eine zentrale Rolle ein. Riboflavin ist in Wasser schlecht löslich, es ist lichtempfindlich, aber sehr hitzestabil. Es sorgt für eine geschmeidige Hautoberfläche und ist an den Reparaturmechanismen der Haut beteiligt.

Die phosphorylierten Vitamin B6-Derivate wirken als Koenzyme in etwa 100 enzymatischen Reaktionen. Fast alle Reaktionen finden im Aminosäurestoffwechsel statt. Eine weitere wichtige Aufgabe übernimmt das Pyridoxalphosphat (PLP oder PALP) (ein Pyridoxin-Derivat) als Cofaktor bei der Synthese der Δ-Aminolävulinsäure, eines Zwischenproduktes in der endogenen Häm-Synthese. Genannt sei auch die Beteiligung von Pyridoxalphosphat als Cofaktor beim Abbau der tierischen Stärke (Glykogen). Mangel bedeutet die Entstehung von Dermatitiden und Wachstumsstörungen. Niacin oder Nicotinsäure ist eine Carbonsäure des Pyridins. Nicotinsäure findet sich in allen lebenden Zellen und wird in der Leber gespeichert. Es bildet einen wichtigen Baustein verschiedener Coenzyme (NAD, NADP) und ist von zentraler Bedeutung für den Stoffwechsel von Eiweißen, Fetten und Kohlenhydraten. Gegenüber Hitze, Licht und dem Luftsauerstoff ist Nicotinsäure weniger empfindlich als andere Vitamine der B-Gruppe. Nicotinsäure ist am Eiweiß-, Fett- und Kohlenhydratstoffwechsel beteiligt. In Form der Coenzyme NAD/NADP und ihrer reduzierten Formen NADH/NADPH, der sogenannten Reduktionsäquivalente, ist die Nicotinsäure z. B. am Citratzyklus und der Atmungskette beteiligt. Sie hat eine antioxidative Wirkung und nimmt an vielen enzymatischen Vorgängen teil. Nicotinsäure ist wichtig für die Regeneration der Haut, Muskeln, Nerven und DNA.

Folsäure ist licht-, Sauerstoff- und hitzeempfindlich sowie gut wasserlöslich. Ein Folsäuremangel im Körper wirkt sich auf das Blutbild aus, indem es zu einer hyperchromen makrozytären Anämie führen kann.

Aufgrund ihrer stoffwechselphysiologischen Eigenschaften haben insbesondere die hier genannten Vitamine - einzeln oder in Kombination - einen erheblichen Einfluß auf die Wundheilung, gerade indem sie die lokale Ernährungssituation verbessern und damit zu einer Verbesserung des lokalen Zellstoffwechsels sorgen.

Besonders bevorzugt ist dabei insbesondere die Kombination aus Vitamin C und Vitamin E. Eine solche Kombination weist besonders synergistische Effekte auf.

Die genannten weiteren Wirkstoffe können durchweg zusammen mit der erfindungsgemäßen Zusammensetzung in eine wasserlösliche Matrix, eine flächige Matte, ein Wunddistanzgitter, eine Schaumstoffmatte, eine Carboxymethylcellulosematte, eine Wundauflage enthaltend superabsorbierende Partikel, eine Lösung, Creme, Salbe, Milch, eine Dispersion, eine Suspension oder ein Gel eingebracht sein.

Erfindungsgemäß ist überdies eine Wundauflage vorgesehen, enthaltend eine Schaummatte und/oder superabsorbierende Partikel, die dadurch gekennzeichnet ist, dass diese eine Zusammensetzung gemäß obiger Beschreibung aufweist.

Ebenso ist eine Wundauflage vorgesehen, enthaltend superabsorbierende Partikel, die dadurch gekennzeichnet, dass diese eine Zusammensetzung gemäß obiger Beschreibung aufweist.

Weiterhin ist ein Kit verschiedener Zusammensetzungen gemäß obiger Beschreibung, verschiedener Matten gemäß obiger Beschreibung oder verschiedener Wundauflagen gemäß obiger Beschreibung vorgesehen, die dadurch gekennzeichnet ist, dass die verschiedenen Zusammensetzungen, Matten oder Wundauflagen des Kits auf jeweils verschiedene Wundphasen abgestimmt sind.

Betrachtet man die bei der Heilung ablaufenden Vorgänge, so lassen sich grob vier Phasen der Wundheilung unterscheiden, nämlich die inflammatorische oder exsudative Phase (Reinigungsphase), die Granulationsphase, die Epithelisationsphase und die reparative Phase. Epithelisationsphase und reparative Phase werden manchmal auch zusammengefasst.

### 1. Reinigungsphase

Insbesondere bei akuten steht unmittelbar nach Entstehen der Wunde die Blutstillung im Vordergrund. Die Komplementkaskade wird aktiviert und der Blutverlust durch Thrombozyten- und Fibrinaggregation begrenzt. Dies wird durch eine gleichzeitige Vasokonstriktion unterstützt. Anschließend kommt es jedoch durch die geschädigten Zellen zu verstärkter Histamin- und Serotoninabgabe. Die daraus resultierende erneute Vasodilatation mit gleichzeitig erhöhter Gefäßpermeabilität führt zur Ausbildung eines Wundödems. Erkennbar ist dies an der Rötung und Erwärmung der Haut sowie den damit einhergehenden Schmerzen des Patienten. Im späteren Teil der Phase steht die Wundreinigung im Vordergrund. Ödematöse Flüssigkeit tritt durch den Wundbereich als proteinreiches Exsudat aus und spült die Wunde. Gleichzeitig wird die Leukozyteninfiltration in den Wundbereich unterstützt. Mehr über Letztlich kommt es zur Wundreinigung durch das mechanische Auswaschen von Keimen und Geweberesten, unterstützt durch biochemische Prozesse zur Keimabwehr und dem aktiven Abbau irreversibel geschädigter Gewebeteile. Die exsudative Phase dient der Vorbereitung des Wundgrundes auf die nachfolgenden Phasen der Wundheilung. Dies gilt insbesondere auch für die Prozesse an chronischen Wunden, die die eingangs erwähnte Blutung nicht durchlaufen haben.

### 2. Granulationsphase

Finden sich in der Wunde durch die exsudative Phase entsprechend vorbereitete Bereiche, so kommt es nach zwei bis vier Tagen zur Einwanderung von Fibroblasten und zur Bildung des Stroma, auch Granulationsgewebe genannt. Die Gewebeneubildung bewegt sich entlang der Fibrinmatrix aus der Blutgerinnung und benötigt die Versorgung mit Nährstoffen durch die zeitgleiche Neubildung von Blutgefäßen (Angiogenese).

### 3. Epithelisationsphase

Durch beginnende Kontraktion der Wundränder wird das Ausmaß der notwendigen Gewebeneubildung reduziert, und die Epithelisierung beginnt, Dabei findet vom Wundrand her eine Gewebeneubildung statt, bei der Keratinozyten oder Basalzellen beteiligt sein können.

### 4. Reparative Phase

In der reparativen Phase wird die Epithelisierung abgeschlossen, sofern ein entsprechend vorbereiteter Wundgrund vorhanden ist. Es kommt zum Umbau des Granulations- in ein Narbengewebe. Dabei werden die Gefäße zurück gebildet, und es entsteht im Laufe von Monaten oder gar Jahren ein hartes, faserreiches Narbengewebe. In dieser Phase kann es insbesondere zum schädlichen Einfluß von Proteasen (insbesondere Matrix-Metalloproteasen) kommen.

Die Wunde weist in jeder Phase unterschiedliche Bedürfnisse auf, welchen durch den erfindungsgemäßen Kit entgegengekommen wird. So kann z.B. ein Kit bestehend aus vier mit unterschiedlichen Zusammensetzungen ausgestatteten Wundauflagen vorgesehen sein. Ein solcher Kit ist in folgender Tabelle beschrieben:

| **Phase** | **Behandlungs und/oder Versorgungsbedarf** | | **Beispielhaft vorzusehende Wirkstoffe in der erfindungsgemäben Zusammensetzung** | |
|---|---|---|---|---|
| | 1. | Aufnahme von Exsudat | 1. | superabsorbierende Partikel |
| | 2. | Hemmung von Bakterienwachstum in der | 2. | in der Wundauflage immobilisierte Silberdonatoren |
| | | Wundauflage | 3. | Gemisch aus Vitaminen bzw. |
| | 3. | Desinfektion der Wunde | | Vitaminderivaten, Metallionen und Tensiden |
| | 4. | Hemmung von Proteasen | 4. | Milchsäurebakterien und Probiotika und/oder TIMP und/oder Säuren |
| 2 | 1. | Nähren der Wunde | 1. | nutritive Wirkstoffe |
| | 2. | Desinfektion der Wunde | 2. | Gemisch aus Vitaminen bzw. |
| | | | | Vitaminderivaten, Metallionen und Tensiden |
| 3 | 1. | Nähren der Wunde | 1. | nutritive Wirkstoffe |
| | 2. | Desinfektion der Wunde | 2. | Gemisch aus Vitaminen bzw. |
| | | | | Vitaminderivaten, Metallionen und Tensiden |
| 4 | 1. | Desinfektion der Wunde | 1. | Gemisch aus Vitaminen bzw. |
| | | | | Vitaminderivaten, Metallionen und Tensiden |
| | 2. | Konditionierung der Wundoberfläche | 2. | Harnstoff, Aloe vera |

Weiterhin ist erfindungsgemäß die Verwendung eines solchen Kits zur äußeren, nicht-systemischen, topischen Versorgung und/oder Behandlung von Wunden des menschlichen oder tierischen Körpers vorgesehen, wobei verschiedene Bestandteile des Kits in verschiedenen Wundphasen verwendet werden.

Abweichend von diesem Ansatz kann natürlich auch vorgesehen sein, eine Zusammensetzung, insbesondere eine Matte, ein Wunddistanzgitter und/oder eine Wundauflage, bereitzustellen, die so eingestellt ist, dass die enthaltenen Wirkstoffe bzw. Wirkstoffkomplexe den Bedürfnissen der Wunde in jeder ihrer Phasen gerecht werden. Hintergrund dieses Ansatzes ist, dass es Wunden gibt, die sich zeitgleich an verschiedenen Stellen in unterschiedlichen Phasen der Wundheilung befinden. Es ist natürlich vorteilhaft, mit nur einem Produkt den Bedürfnissen all dieser Stellen gerecht werden zu können.

Ebenso kann z.B. das Löslichkeitsverhalten einzelner Wirkstoffe bzw. Wirkstoffkomplexe so eingestellt sein, dass sie zu unterschiedlichen Zeitpunkten (d. h. in unterschiedlichen Phasen der Wundheilung) gelöst und der Wunde zugeführt werden. Hierzu kann z. B. die Partikelgröße unterschiedlich eingestellt sein, d. h. bei spät verfügbar zu machenden Wirkstoffen bzw. Wirkstoffkomplexen kann eine große und bei früh verfügbar zu machenden Wirkstoffen bzw. Wirkstoffkomplexen eine niedrige Partikelgröße eingestellt sein (Depotwirkung).

Alternativ können die jeweiligen Wirkstoffe bzw. Wirkstoffkomplexe in abbaubaren Kapseln mit unterschiedlichen Abbaukonstanten angeordnet sein, um so der Wunde in den jeweils zutreffenden Phasen zugeführt zu werden. Auch auf diese Weise kann also eine Depotwirkung herbeigeführt werden.

Zusammenfassend ist zu sagen, dass durch diese Arten der Wundphasengerechten Versorgung neue Maßstäbe in der Wundbehandlung gesetzt werden können.

### Zeichnungen und Beispiele

Die vorliegende Erfindung wird durch die im Folgenden gezeigten und diskutierten Figuren und Beispiele genauer erläutert. Dabei ist zu beachten, dass die Figuren und Beispiele nur beschreibenden Charakter haben und nicht dazu gedacht sind, die Erfindung in irgendeiner Form einzuschränken.

### Beispiel 1: Zusammensetzung einer erfindungsgemäßen nutritiven Zusammensetzung ohne Proteine

| **Bestandteil** | **Menge** |
|---|---|
| Isoleucin | 2,5 g |
| Leucin | 3,7 g |
| Lysin-HCl | 4,125 g |
| Methionin | 2,15 g |
| Phenylalanin | 2,55 g |
| Threonin | 2,2 g |
| Tryptophan | 1 g |
| Valin | 3,1 g |
| Arginin | 6 g |
| Histidin | 1,5 g |
| Glycin | 7 g |
| Alanin | 7,5 g |
| Prolin | 7,5 g |
| Äpfelsäure | 3,065 g |
| Glucose 1H₂O | 220 g |
| Natriumchlorid | 1,169 g |
| Kaliumchlorid | 2,238 g |
| Calciumchlorid 2H₂O | 0,368 g |
| Magnesiumchlorid 6H₂O | 0,509 g |
| Zinkchlorid | 0,0055 g |
| Glycerol-1(2)-dihydrogenphosphat - Gemisch der Dinatriumsalze | 4,592 g |
| Summe | 282,78 |

Diese Zusammensetzung entspricht derjenigen eines vollbilanzierten parenteralen Diätetikums. Sie deckt in der genannten Menge etwa 60 % des Tagesbedarfs eines 80 kg schweren Mannes entsprechend der von der DGE (Deutsche Gesellschaft für Ernährungsmedizin) empfohlenen Tagesdosierungen. Bei der Verwendung in einem Wundauflageprodukt liegt hier also eine topische Überdosierung vor. Diese Überdosierung dient jedoch nicht dem Ziel, eine allgemeine Nährstoffmangelsitutation eines Patienten zu beheben oder eine systemische Sepsis zu unterbinden, da eine systemische Wirkung wie oben erwähnt weder angestrebt noch erwünscht ist.

### Beispiel 2: Zusammensetzung einer erfindungsgemäßen nutritiven Zusammensetzung ohne Proteine und ohne Kohlenhydratquelle

Isoleucin 2,5 g, Leucin 3,7 g, Lysin-HCl 4,125 g (entspr. 3,3 g L-Lysin), Methionin 2,15 g, Phenylalanin 2,55 g, Threonin 2,2 g, Tryptophan 1 g, Valin 3,1 g, Arginin 6 g, Histidin 1,5 g, Glycin 7 g, Alanin 7,5 g, Prolin 7,5 g, Äpfelsäure 3,065 g, Natriumchlorid 1,169 g, Kaliumchlorid 2,238 g, Calciumchlorid 2H₂O 0,368 g, Magnesiumchlorid 6H₂O 0,509 g, Zinkchlorid 0,0055 g, Glycerol-1(2)-dihydrogenphosphat - Gemisch der Dinatriumsalze 5H₂O (40/60 G/G) 4,592 g (entspr. mmol/l: Na⁺ 50, K⁺ 30, Ca²⁺ 2,5, Mg²⁺ 2,5, Zn²⁺ 0,04, Cl⁻ 100,11, Glycerophosphat 15).

Diese Zusammensetzung entspricht derjenigen eines vollbilanzierten parenteralen Diätetikums unter Auslassung von Glucose. Dies kann bei hochinfizierten Wunden angezeigt sein, um eine Kohlenhydratversorgung der infektiösen Bakterien zu unterbinden.

### Beispiel 3. Zusammensetzung einer weiteren erfindungsgemäßen nutritiven Zusammensetzung

Glucosesirup, Maltodextrin, Pflanzliche Öle, Sojaproteinisolat, Milcheiweiß, Inulin, Guarkernmehl, Glucose, Sojafaser, Emulgator: Mono- und Diglyceride von Speisefettsäuren, Kaliumcitrat, Natriumchlorid, Emulgator: Sojalecithin, Magnesiumcarbonat, Calciumorthophosphate, Kaliumchlorid, Cholinhydrogentartrat, Calciumcarbonat, Vitaminmischung (Vitamin C, Niacin, Vitamin E, Pantothenat, Vitamin B2, Vitamin B6, Vitamin B1, Vitamin A, Folsäure, Vitamin K, Biotin, Vitamin D3, Vitamin B12), Natriumcitrat, Taurin, Eisenlactat, L-Carnitin, Zinkoxid, Kaliumfluorid, Mangansulfat, Kupfersulfat, Kaliumjodat und Chromchlorid.

Die besagte Zusammensetzung weist folgende Nährstoffgehalte auf:

| **Komponente** | **⌀ Gehalt pro 100g Pulver** |
|---|---|
| Energie | 1821 kJ/ 433 kcal |
| Protein (13 % Energie) | 13,8 g |
| Kohlenhydrate (56 % E.) | 60 g |
| Ballaststoffe | 6,2 g |
| Fett (30 % Energie) | 14,6 g |
| - gesättigte Fettsäuren | 5,2 g |
| - einfach ungesättigte Fettsäuren | 6,4 g |
| - mehrfach ungesättigte Fettsäuren | 3 g |
| Natrium | 260 mg |
| Kalium | 435 mg |
| Calcium | 260 mg |
| Magnesium | 70 mg |
| Phosphor | 175 mg |
| Chlorid | 385 mg |
| Eisen | 4,8 mg |
| Zink | 4,7 mg |
| Kupfer | 435 µg |
| Jod | 45 µg |
| Chrom | 20 µg |
| Fluorid | 0,4 mg |
| Mangan | 0,6 mg |
| Molybdän | 22 µg |
| Selen | 12,5 µg |
| Vitamin A | 260 µg |
| Vitamin D | 3,1 µg |
| Vitamin E | 9,5 mg |
| Vitamin K | 35 µg |
| Vitamin B1 | 0,45 mg |
| Vitamin B2 | 0,58 mg |
| Vitamin B6 | 0,45 mg |
| Vitamin B12 | 1,1 µg |
| Vitamin C | 60 mg |
| Niacin | 5,9 mg |
| Folsäure | 70 µg |
| Pantothensäure | 2,6 mg |
| Biotin | 16 µg |
| Cholin | 77 mg |
| Taurin | 24 mg |
| L-Carnitin | 6,5 mg |
| Inositol | 18 mg |

Diese Zusammensetzung entspricht derjenigen eines vollbilanzierten enteralen Diätetikums. Sie deckt in den genannten Mengen etwa 30 % des Tagesbedarfs eines 80 kg schweren Mannes entsprechend der von der DGE (Deutsche Gesellschaft für Ernährungsmedizin) empfohlenen Tagesdosierungen. Bei der Verwendung in einem Wundauflageprodukt liegt hier also eine topische Überdosierung vor. Diese Überdosierung dient jedoch nicht dem Ziel, eine allgemeine Nährstoffmangelsitutation eines Patienten zu beheben oder eine systemische Sepsis zu unterbinden, da eine systemische Wirkung wie oben erwähnt weder angestrebt noch erwünscht ist.

Es kann ggf. vorgesehen sein, den Kohlenhydratanteil (insbesondere Glucose, aber ggf. auch Maltose, Maltodextrin, Isomaltose oder Stärke) wegzulassen, um bei hochinfizierten Wunden eine Kohlenhydratversorgung der infektiösen Bakterien zu unterbinden. Ebenso kann vorgesehen sein, Ballaststoffe wie Inulin oder Sojafaser, Emulgatoren wie Mono- und Diglyceride oder Sojalecithin und/oder Verdickungsmittel wie Guarkernmehl wegzulassen.

**Beispiel 4:** Zusammensetzung verschiedener erfindungsgemäßer, desinfizierend wirkender Zusammensetzungen enthaltend mindestens ein Vitamin oder Vitaminderivat, ein Metallion und ein Detergens in 50 µl. destilliertem Wasser.

| **Vitamin bzw.-derivat** | **Metallion** | **Detergenz** |
|---|---|---|
| 100 mM Vitamin C | 10 mM FeCl₃ | 0,5 % SDS |
| 50 mM Vitamin E | | |
| 100 mM Vitamin C 50 | 10 mM ZnCl₂ | 2 % TritonX-100 |
| mM Vitamin E | | 0,2 % Tween 20 |

### Beispiel 5: erfindungsgemäße Zusammensetzung enthaltend nicht pathogene, milchsäureproduzierende Mikroorganismen als ansäuernd wirkende Wirkelemente

Eine Zusammensetzung enthaltend folgende Bestandteile:

| **Bestandteil** | **Menge** |
|---|---|
| *Lactobacillus acidophilus* | 6,3 Gew.-% |
| *Lactococcus lactis* | 2,1 Gew.-% |
| *Bifidobacterium longum* | 2,1 Gew.-% |
| *Lactobacillus rhamnosus* | 1,4 Gew.-% |
| *Bifidobacterium breve* | 1,4 Gew.-% |
| *Bifidobacterium bifidum* | 0,7 Gew.-% |
| Ascorbinsäure | 1 Gew.-% |
| Kartoffelstärke als Träger | ad 100 Gew.-% |

Die genannten Mikroorganismen liegen in gefriergetrockneter Form vor und werden bei Kontakt mit der Wunde durch Wärme und Feuchtigkeit aktiviert.

Die Zusammensetzung ist so eingestellt, dass 2g derselben mindestens jeweils 5x10⁸ der betreffenden Keime enthalten.

### Beispiel 6: erfindungsgemäße Zusammensetzung enthaltend nicht pathogene, milchsäureproduzierende Mikroorganismen als ansäuernd wirkende Wirkelemente sowie Präbiotika

Zu 2 g der Zusammensetzung aus Beispiel 5 werden 5g eines Gemischs aus Inulin (65 Gew.-%) Oligofructose (20 Gew.-%) und Topinambursaft-Pulver (15 Gew.-%) gegeben.

### Beispiel 7: Herstellung einer SAP enthaltenden Wundauflage enthaltend eine erfindungsgemäße Zusammensetzung

Eine Zusammensetzung gemäß einem der Beispiele 1 - 6 wird zusammen mit Zellulosefasern verpresst. Dabei werden folgende Mengen pro 100 g Zellulosefasern verwendet:

| **Zusammensetzung aus Beispiel** | **Menge** | **Entspricht Anteil an empfohlener systemischer Tagesdosis von** |
|---|---|---|
| 1 (nutritiv) | 100 g | 21 % |
| 2 (nutritiv) | 22 g | 21 % |
| 3 (nutritiv) | 100 g | 30% |
| 4 (desinfizierend) | 50 µl Wasser | n/n |
| | 100 mM Vitamin C 50 mM Vitamin E 10 mM FeCl₃ 0,5 % SDS | |
| 5 (ansäuernd) | 2g | 100 % |
| 6 (Proteasen hemmend) | 7 g | 100 % |
| 1 + 4 (nutritiv und desinfizierend, Geeignet für Wundphase 3) | s.o. | 21 % |

Die Zellulosefasern werden dann mit einem Verfahren, wie es z.B. in der DE19750890 beschrieben ist, deren Unhalt dem Offenbarungsgahelt dieser Schrift vollumfänglich hinzugefügt werden soll, mit 50 Gew.-% superabsorbierender Polymere (Copolymer aus Acrylsäure und Natriumacrylat) versetzt und zu einem Airlaid mit einer Größe von 20 x 10 cm verarbeitet. Anschließend wird dieses Airlaid beidseitig mit einer Zellulosematte versehen und in einer Hülle aus Polypropylen verpackt, die Poren mit einer Größe zwischen 0,1 mm und 1,0 mm aufweist. Die Hülle wird an den Rändern mit einer Ultraschallnaht versehen.

### Beispiel 8: Herstellung einer Wundauflage aus einem PU-Schaum enthaltend eine erfindungsgemäße Zusammensetzung.

Eine Zusammensetzung gemäß einem der Beispiele 1 - 6 wird in derselben Menge wie in Beispiel 7 in 200 ml destilliertem Wasser gelöst. Anschließend wird eine 20 x 20 x 0,3 cm messende Matte aus PU-Schaum mit dieser Lösung getränkt und getrocknet.

### Beispiel 9: Herstellung einer Wundauflage enthaltend ein QAV

Eine Zusammensetzung gemäß einem der Beispiele 1 - 6 sowie 20 mg Benzalkoniumchlorid werdwn in derselben Menge wie in Beispiel 7 in 200 ml destilliertem Wasser gelöst. Anschließend wird eine 20 x 20 x 0,3 cm messende Matte aus PU-Schaum mit dieser Lösung getränkt und getrocknet.

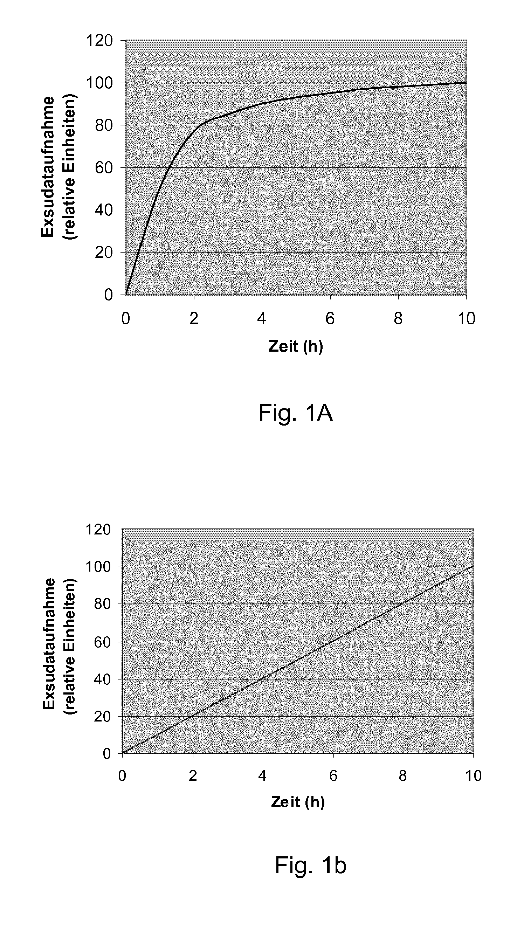

**Fig. 1****: Aufnahme von Exsudaten durch eine im Wesentlichen trockene bzw. eine benetzte superabsorbierende Polymere enthaltende Wundauflage**

Fig. 1A zeigt die Exsudataufnahme einer im Wesentlichen trockenen, absorbierenden Wundauflage aufweisend superabsorbierende Polymere, wie sie z.B. in der DE10059439 sowie der WO03094813 der Anmelderin der vorliegenden Erfindung beschrieben ist. Eine solche Wundauflage weist eine gerade in der Anfangsphase enorm hohe Aufnahmekapazität auf, die sich in dem gezeigten hyperbolischen Verlauf darstellt. Aufgrund der Bindungseigenschaften der superabsorbierenden Poylmere gegenüber Proteinen, insbesondere Matrixproteasen, sowie zweiwertigen Kationen, werden dieses sehr schnell aus der Wunde abgeführt, und der Heilungsprozess damit gefördert. Insbesondere ist dies von Vorteil, wenn die Wundauflagen relativ häufig gewechselt werden. Es ist dann sichergestellt, dass zum Zeitpunkt des Wechsels die Sättigungskapazität der Wundauflage (und damit die maximale Bindungsfähigkeit für Matrixproteasen und zweiwertigen Kationen) bereits erreicht ist.

Fig. 1B zeigt hingegen die Exsudataufnahme einer mit Ringerlösung benetzten, superabsorbierende Polymere enthaltenden Wundauflage. Hierbei handelt es sich um einen Spülkörper, der auf eine Wunde aufgelegt wird, in einem zyklischen Prozess Ringerlösung an die Wunde abgibt und wieder aufnimmt und dabei die Wunde spült. Eine solche Wundauflage weist also keine Nettoaufnahme an Flüssigkeit auf. Während des Spülprozesses wird hingegen die Wunde sukzessive ausgewaschen und Exsudat sowie dessen Inhaltsstoffe, insbesondere Matrixproteasen und zweiwertige Kationen, in die Wundauflage verbracht, wo sie durch die dort befindlichen superabsorbierenden Polymere gebunden werden. Aufgrund des passiven Spülprinzips ist dies jedoch ein sehr langwieriger Vorgang, der mitunter eine längere Zeit benötigt als die mittlere Verweildauer der Wundauflage auf der Wunde beträgt. Es kann also vorkommen, dass die Wundauflage bereits zu einem Zeitpunkt ausgewechselt wird, an welchem sie die maximale Sättigung noch nicht erreicht hat.

## Patentansprüche

1. Zusammensetzung, enthaltend mindestens einen nutritiven, mindestens einen desinfizierenden bzw. dekontaminierenden und/oder mindestens einen Proteasen hemmend wirkenden Wirkstoff und/oder Wirkstoffkomplex zur äußeren Versorgung und/oder Behandlung von Wunden des menschlichen oder tierischen Körpers.

2. Verwendung einer oder mehrerer Zusammensetzungen gemäß Anspruch 1 zur Herstellung eines Mittels zur äußeren, nicht-systemischen, topischen Versorgung und/oder Behandlung von Wunden des menschlichen oder tierischen Körpers.

3. Zusammensetzung gemäß Anspruch 1 bzw. Verwendung gemäß Anspruch 2, **dadurch gekennzeichnet, dass** die Konzentration und/oder die Darreichungsform des mindestens einen Wirkstoffs oder Wirkstoffkomplexes so gewählt ist, dass er lediglich eine topische, nicht systemische Wirkung entfaltet.

4. Zusammensetzung bzw. Verwendung gemäß einem der vorherigen Ansprüche, **dadurch gekennzeichnet, dass** der mindestens eine Wirkstoff oder Wirkstoffkomplex in wasserlöslicher Form vorliegt.

5. Zusammensetzung bzw. Verwendung gemäß einem der vorherigen Ansprüche, **dadurch gekennzeichnet, dass** der mindestens eine Wirkstoff oder Wirkstoffkomplex in einer Form vorliegt, die nach mittelbarer oder unmittelbarer Aufbringung derselben auf die Wunde eine Migration derselben in die Wunde ermöglicht.

6. Zusammensetzung bzw. Verwendung gemäß einem der vorherigen Ansprüche, **dadurch gekennzeichnet, dass** der mindestens eine Wirkstoff oder Wirkstoffkomplex in eine wasserlösliche Matrix eingebracht ist.

7. Zusammensetzung bzw. Verwendung gemäß einem der vorherigen Ansprüche, **dadurch gekennzeichnet, dass** der mindestens eine Wirkstoff oder Wirkstoffkomplex in eine flächige Matte eingebracht ist.

8. Zusammensetzung bzw. Verwendung gemäß einem der vorherigen Ansprüche, **dadurch gekennzeichnet, dass** der mindestens eine Wirkstoff oder Wirkstoffkomplex in ein Wunddistanzgitter eingebracht ist.

9. Zusammensetzung bzw. Verwendung gemäß einem der vorherigen Ansprüche, **dadurch gekennzeichnet, dass** der mindestens eine Wirkstoff oder Wirkstoffkomplex in eine absorbierende Wundauflage aufweisend mindestens ein Wirkelement ausgewählt aus der Gruppe enthaltend eine Schaumstoffmatte, ein Airlaid, eine Carboxymethylcellulosematte, eine Alginatmatte und/oder eine Matte aufweisend superabsorbierende Partikel eingebracht ist.

10. Zusammensetzung bzw. Verwendung gemäß einem der vorherigen Ansprüche, **dadurch gekennzeichnet, dass** der mindestens eine Wirkstoff oder Wirkstoffkomplex in eine Wundauflage enthaltend superabsorbierende Partikel sowie mindestens eine Schaumstoffmatte und/oder eine Carboxymethylcellulosematte eingebracht ist.

11. Zusammensetzung bzw. Verwendung gemäß einem der vorherigen Ansprüche, **dadurch gekennzeichnet, dass** der mindestens eine Wirkstoff oder Wirkstoffkomplex in eine Lösung eingebracht ist.

12. Zusammensetzung bzw. Verwendung gemäß einem der vorherigen Ansprüche, **dadurch gekennzeichnet, dass** der mindestens eine Wirkstoff oder Wirkstoffkomplex in eine Creme, Salbe, Milch, Emulsion, Suspension, Dispersion oder ein Gel eingebracht ist.

13. Zusammensetzung bzw. Verwendung gemäß einem der vorherigen Ansprüche, **dadurch gekennzeichnet, dass** mindestens ein Wirkstoff in einer durch ein Verfahren ausgewählt aus der Gruppe enthaltend Gefriertrocknen, Lyophilisation, Sprühtrocknen, Walzentrocknen und/oder Vakuumverdampfen aufbereiteten Form vorliegt.

14. Zusammensetzung bzw. Verwendung gemäß einem der vorherigen Ansprüche, **dadurch gekennzeichnet, dass** die Zusammensetzung bzw. ihre Bestandteile in sterilisierter Form vorliegen.

15. Zusammensetzung bzw. Verwendung gemäß einem der vorherigen Ansprüche, **dadurch gekennzeichnet**, bei es sich bei mindestens einem der desinfizierend wirkenden Wirkstoffkomplexe um eine Zusammensetzung aus mindestens einem Vitamin oder Vitaminderivat, einem Metallion sowie einem Detergenz handelt.

16. Zusammensetzung bzw. Verwendung gemäß einem der vorherigen Ansprüche, **dadurch gekennzeichnet, dass** es sich bei mindestens einem der desinfizierend wirkenden Wirkstoffe bzw. Wirkstoffkomplexe um einen BLIS (bacteriocin like inhibitory substance) handelt.

17. Zusammensetzung bzw. Verwendung gemäß einem der vorherigen Ansprüche, **dadurch gekennzeichnet, dass** es sich bei mindestens einem der desinfizierend wirkenden Wirkstoffe bzw. Wirkstoffkomplexe um beschichtete magnetische Partikel handelt.

18. Zusammensetzung bzw. Verwendung gemäß einem der vorherigen Ansprüche, **dadurch gekennzeichnet, dass** es sich bei mindestens einem der nutritiv wirkenden Wirkstoffkomplexe um eine Zusammensetzung enthaltend mindestens die Bestandteile eines enteralen und/oder parenteralen Diätetikums handelt.

19. Zusammensetzung bzw. Verwendung gemäß einem der vorherigen Ansprüche, **dadurch gekennzeichnet, dass** es sich bei mindestens einem der nutritiv wirkenden Wirkstoffe bzw. Wirkstoffkomplexe um mindestens ein Wirkelement ausgewählt aus der Gruppe enthaltend Insulin, rekombinantes Insulin, Proinsulin, einen insulinähnlichen Wachstumsfaktor (Insulin-like growth factor, IGF), ein Insulinmimetikum und/oder einen diabetikerspezifischen, nicht glucose- bzw. saccharosebasierenden Energieträger handelt.

20. Zusammensetzung bzw. Verwendung gemäß einem der vorherigen Ansprüche, **dadurch gekennzeichnet, dass** es sich bei mindestens einem der Proteasen hemmend wirkenden Wirkstoffe und/oder Wirkstoffkomplexe um mindestens ein Wirkelement ausgewählt aus der Gruppe enthaltend Proteasehemmer, superabsorbierende Polymere, Chelatoren für zweiwertige Kationen, Kollagen, beschichtete magnetische Partikel, Säuren, Puffer, nicht pathogene säureproduzierende Mikroorganismen, Probiotika und/oder Symbiotika handelt.

21. Zusammensetzung bzw. Verwendung gemäß einem der vorherigen Ansprüche, **dadurch gekennzeichnet, dass** die Zusammensetzung überdies einen oder mehrere Wirkstoffe enthält, die ausgewählt sind aus der Gruppe enthaltend orthomolekulare Nährstoffe, Nutraceuticals, Phytochemicals, Wachstumsfaktoren, Erdölbasierende Zugpräparate. Methyxanthine, Mineralokortikoide, Gerbstoffe, Tacrolimus, Pinecrolimus, Polidocanol, Tenside, ATP, Harnstoff, Nekrolytika, Sympathomimetika, Parasympatholytika, Aktivkohle, Octenidin, Polihexanid, Homöopathika, Q10, Lysin, Pektin, Agar, Aloe vera, Hämostyptika, Ameisensäure, Fruchtsäure, Bernsteinsäure, Madenspeichel, Spinnwebenproteine, Kollagen, Glycerin, Biofilm beschädigende Substanzen, Triacetin, Dickungsmittel, Karaja, Zinkoxid, geruchshemmende Stoffe, Gelbildner, exsudationsfördernde Stoffe und/oder abschwellend wirkende Stoffe.

22. Zusammensetzung bzw. Verwendung gemäß einem der vorherigen Ansprüche, **dadurch gekennzeichnet, dass** es sich bei den Vitaminen um ein oder mehrere Vitamine handelt, die ausgewählt sind aus der Gruppe enthaltend Vitamin B12, Vitamin D, Vitamin C, Vitamin B1, Vitamin B2, Vitamin B6, Niacin und/oder Folsäure.

23. Wundauflage, enthaltend eine Schaummatte und/oder superabsorbierende Partikel, **dadurch gekennzeichnet, dass** diese eine Zusammensetzung gemäß einem der Ansprüche 1 - 22 aufweist.

24. Kit verschiedener Zusammensetzungen gemäß einem der Ansprüche 1 - 22, Matten gemäß Anspruch 7, Wunddistanzgitter gemäß Anspruch 8 und/oder Wundauflagen gemäß einem der Ansprüche 9 - 12 bzw. 23, **dadurch gekennzeichnet, dass** die verschiedenen Bestandteile des Kits auf jeweils verschiedene Wundphasen abgestimmt sind.

25. Verwendung eines Kits gemäß Anspruch zur äußeren, nicht-systemischen, topischen Versorgung und/oder Behandlung von Wunden des menschlichen oder tierischen Körpers, wobei verschiedene Bestandteile des Kits in verschiedenen Wundphasen verwendet werden.
